# EUROPEAN PATENT APPLICATION

(11) **EP 4 711 449 A1**
(43) Date of publication of application: **18.03.2026**
(21) Application number: 24807162.3
(22) Date of filing: 10.05.2024
(51) Int. Cl.: C12N 5/10, C12N 5/074, C12N 5/0735, C12N 15/12

(54) **PROLIFERATION ACTIVATION OF PANCREATIC ISLET CELLS BY ACTIVATION OF GLUCOSE PATHWAY**

(30) Priority: 12.05.2023 JP 2023079704
(71) Applicant: The University of Tokyo, Bunkyo-ku, Tokyo 113-8654 (JP)
(72) Inventor: YAMADA, Yasuhiro, Tokyo 113-8654 (JP); HIRANO, Michitada, Tokyo 113-8654 (JP)
(74) Representative: Kransell & Wennborg KB
(86) International application number: PCT/JP2024/017478
(87) International publication number: WO 2024/237210

(57) **Abstract**

The purpose of the present invention is to provide a method that sustains proliferation of mature pancreatic islet cells by a Mycl gene and enables continuous induction of proliferation. The present invention provides a method for promoting and/or sustaining, by a glucose stimulation activation pathway activator, the proliferation activity of pancreatic islet cells in which a Mycl gene or a gene product thereof has been introduced or expressed through induction. More specifically, in the method, the Mycl gene includes (1) a nucleic acid having a base sequence represented by SEQ ID NO: 1 or 3, or (2) a nucleic acid that hybridizes with, under stringent conditions, a nucleic acid having a base sequence represented by SEQ ID NO: 1 or 3, and that encodes a polypeptide having the function of sustaining and/or promoting the proliferation activity of pancreatic islet-like cells when expression of the Mycl gene is induced.

## Description

### FIELD

The present invention relates to a method for sustaining and promoting proliferation of pancreatic islet cells that have the Mycl gene or its gene product transferred or have its expression induced, by regulating a pathway activated by glucose.

### BACKGROUND

Worldwide medical costs for diabetes and its complications are said to have reached 90 trillion yen per year, and medical expenses related to it have become a major problem for society. Diabetes causes complications such as nephropathy in addition to retinopathy and neuropathy, with artificial dialysis often being necessary in the late stages. For severe end-stage diabetes cases in which almost no insulin secretion is observed, blood glucose control becomes especially poor and results in bouts of severe hypoglycemia that can potentially lead to coma or death. Since severe hypoglycemia leads to after-effects such as brain damage including cognitive disorders, as well as general poor prognosis, it has become an issue of clinical importance to avoid onset of severe hypoglycemia. Treatment methods for diabetes generally involve administration of drugs that inhibit sugar absorption or symptomatic therapy such as administration of insulin formulations, but virtually none focus on curing diabetes itself.

Pancreatic transplant or pancreatic islet transplant have been carried out for patients with poor prognosis and particularly poor blood sugar control, with depleted insulin secretion and suffering from repeated bouts of severe hypoglycemia, and such procedures have had marked effects including not only inhibition of severe hypoglycemia but also providing insulin independence and satisfactory glycemic control. However, the number of transplant receiving patients is extremely limited due to a lack of donors and few facilities available for separation of grafting pancreatic islets, and therefore pancreatic transplant or pancreatic islet transplant have not been provided as early intervention treatment options for patients having only a history of severe hypoglycemia, for high-risk groups with depleted insulin secretion, or ordinary type I diabetes patients. In 2020, donor pancreatic islet transplantation was listed as being covered by insurance under the description: "allogeneic corpse pancreatic islet transplantation". While treatment results have improved over the years, issues remain not only because of a lack of donors, but also in terms of ensuring the quality and yield of pancreatic islets due to individual differences in donor pancreatic islets and differences in timing and methods up until separation of the pancreatic islets. These issues have created a problematic situation in which a limited number of patients are suitable for grafting, scheduled pancreatic islet transplant operations are canceled, and repeated transplantation is necessary to exhibit sufficient therapeutic effects such as insulin independence. Despite advances in cell transplantation medicine using pluripotent stem cell-derived insulin-producing cells, a major obstacle is that pluripotent stem cell-derived insulin-producing cells are functionally immature. Moreover, due to the complexity of the differentiation-inducing step, it is currently difficult to induce differentiation from pluripotent stem cells to endoderm, pancreatic tissue and pancreatic islets, in an economical manner.

As an alternative, it is expected that proliferation of a sufficiently large amount of donor pancreatic islet cells should allow safe, high-quality products to be stably supplied at relatively low cost by relatively convenient means of managing production steps and quality. Furthermore, since this makes it possible to graft high-quality pancreatic islet cells in an adequate amount, a sufficient therapeutic effect can potentially be exhibited by a single graft operation. In addition, transplantation therapy can be carried out in a systematic manner by cryopreservation of the intermediate product or final product, thus helping to minimize the risk of canceled transplantation resulting from unstable pancreatic islet supplies. Guaranteeing stable supply of a sufficient amount of grafting pancreatic islets is expected to widen the range of patients for whom such treatment can be implemented. Causing proliferation of pancreatic islet cells derived from pluripotent stem cells may also be expected to lower costs for production of pluripotent stem cell-derived pancreatic islet cells for transplantation. This is likely to have a major impact in treatment strategies for diabetes compared to the existing treatment methods. In recent years, inducing proliferation of mature pancreatic islet cells has become possible by transfer of the Mycl gene. This has raised the prospect of a new strategy of transplant medicine by inducing proliferation of pancreatic islet cells using Mycl (PTL 1). However, causing proliferation of mature pancreatic islet cells by transfer of the Mycl gene is still limited, with limited amplification efficiency as an associated problem.

### [CITATION LIST]

### [PATENT LITERATURE]

[PTL 1] International Patent Publication No. WO2021/117840

### SUMMARY

### [TECHNICAL PROBLEM]

While expression of the Mycl gene has allowed proliferative activity to be imparted to mature pancreatic islet cells in the prior art, the pancreatic islet growth is only temporary, with proliferative activity declining after repeated subcultures, and this makes it impossible to accomplish continuous amplification. It is therefore an object of the present invention to provide a method that can continuously induce sustained proliferation of mature pancreatic islet cells by the Mycl gene.

### [SOLUTION TO PROBLEM]

The present inventors have completed this invention upon finding that it is possible to cause proliferation of pancreatic islet cells by regulating glucose-activated pathways, which includes activating proliferation of mature pancreatic islet cells by the Mycl gene in a glucose concentration-dependent manner, using a glucokinase activator to activate proliferation of mature pancreatic islet cells by the Mycl gene, and repeating short-term stimulation with high-concentration glucose, while also making it possible to amplify mature pancreatic islet cells for prolonged periods.

Specifically, the invention has the following aspects.
[1] A method of promoting and/or sustaining proliferation activity for pancreatic islet cells into which the Mycl gene or its gene product has been transferred or where its expression has been induced, using an activator of the glucose-stimulated activation pathway.
[2] The method according to [1], wherein the activator of the glucose-stimulated activation pathway is at least one selected from the group consisting of glucose, sucrose and glucokinase activators.
[3] The method according to [1] or [2], wherein the Mycl gene comprises:
   (1) a nucleic acid comprising the nucleotide sequence represented by SEQ ID NO: 1 or 3; or
   (2) a nucleic acid encoding a polypeptide which hybridizes with a nucleic acid comprising the nucleotide sequence represented by SEQ ID NO: 1 or 3 under stringent conditions, and which has a function of sustaining and/or promoting the proliferative activity of pancreatic islet-like cells in a glucose concentration-dependent manner when expression of the Mycl gene has been induced.
[4] The method according to [1] or [2], wherein the Mycl gene product comprises:
   (1) a polypeptide comprising the amino acid sequence represented by SEQ ID NO: 2 or 4; or
   (2) a polypeptide having at least 80%, 85%, 90%, 95%, 97%, 98% or 99% sequence identity with the amino acid sequence represented by SEQ ID NO: 2 or 4, and having the function of sustaining and/or promoting the proliferative activity of pancreatic islet-like cells in a glucose concentration-dependent manner.
[5] The method according to any one of [1] to [4], wherein the Mycl gene is transiently expressed.
[6] The method according to any one of [1] to [5], wherein high-concentration glucose and low-concentration glucose are alternately applied.
[7] The method according to any one of [1] to [6], wherein the pancreatic islet cells are derived from primary islet cells isolated from pancreas, cultured islet cells or stem cells.
[8] The method according to [7], wherein the stem cells are selected from the group consisting of iPS cells, ES cells and somatic stem cells.

### [ADVANTAGEOUS EFFECTS OF INVENTION]

According to the invention it is possible to treat diabetes or its associated conditions in which insulin production is desired, by regulating the glucose-activated pathway to cause proliferation of pancreatic islet cells and maintain proliferative activity of pancreatic islet-like cells for prolonged periods.

### BRIEF DESCRIPTION OF DRAWINGS

Fig. 1 shows activation of Mycl-induced pancreatic islet cell proliferation in a test with glucose.
Fig. 2 shows glucose concentration-dependent activation of Mycl-induced pancreatic islet cell proliferation.
Fig. 3 shows changes in efficiency of Mycl-induced pancreatic islet cell proliferation by a glucokinase activator (GKA).
Fig. 4 shows changes in efficiency of Mycl-induced pancreatic islet cell proliferation by a glucokinase inhibitor (GKI).
Fig. 5 shows activation of Mycl-induced pancreatic islet cell proliferation with glucose *in vivo.*
Fig. 6 shows reduction in Mycl-induced pancreatic islet cell proliferation activity under culturing conditions with high glucose concentration.
Fig. 7 shows sustained Mycl-induced pancreatic islet cell proliferation activity with High-Low glucose.
Fig. 8 shows sustained Mycl-induced pancreatic islet cell proliferation activity with High-Low glucose.
Fig. 9 shows promotion of proliferation of mature hormone-producing cells by Mycl gene expression.
Fig. 10 shows efficient induction of β cell proliferation by coordination of Mycl gene expression and glucose.
Fig. 11 shows restoration of diabetic mice by prolonged culturing of pancreatic islet cells.

### DESCRIPTION OF EMBODIMENTS

The present invention relates to a method of sustaining and/or promoting proliferative activity of pancreatic islet cells having the Mycl gene or its gene product transferred or having its expression induced (hereunder also referred to as "pancreatic islet-like cells"), in a glucose concentration-dependent manner. The present invention will now be explained in greater detail.

### 1. Pancreatic islet-like cells and preparation method

### (1) Pancreatic islet-like cells

The term "pancreatic islet-like cells" as used herein refers to mature pancreatic islet cells in which the Mycl gene or its gene product has been transferred. Throughout the present specification, pancreatic islet-like cells that have moved to the growth phase by forced expression of the Mycl gene will be referred to as "pancreatic islet precursor-like cells". Stopping expression of the gene can cause differentiation of pancreatic islet cells that include cells capable of producing insulin (hereunder also referred to as "insulin-producing cells"). In other words, the present invention can increase the number of pancreatic islet-like cells by transient expression of the Mycl gene, and cause them to differentiate into pancreatic islet cells including insulin-producing cells. Focusing on markers expressed by different cells, "pancreatic islet precursor-like cells" are positive for at least one, preferably two and more preferably three or more genes selected from among Fev, Pax4, Cck, CDK4 and Ki67, for example. Focusing on the expressed proteins, they are characterized by reduced production of insulin and glucagon in particular compared to normal pancreatic islets, or by notable somatostatin production.

### (2) Pancreatic islet cells

"Pancreatic islet cells" generally refers to cell aggregates known as islets of Langerhans which perform pancreatic endocrine functions, as endocrine cells making up about 1 to 2% of all of the cells in the pancreas. As used herein, the term "pancreatic islet cells" is interchangeable with "mature pancreatic islet cells". Pancreatic islet cells are largely composed of five different types of cells: α cells, β cells, δ cells, ε cells and PP cells. The major cells composing cell aggregates known as "pancreatic islets" are β cells. The β cells constitute about 60 to 80% of pancreatic islet cells and secrete insulin which aids in migration of glucose into most cells in the body. On the other hand, α cells constitute approximately 10 to 30% of pancreatic islets, secreting glucagon which is released during fasting, whereby glycogen stored in the liver is decomposed to maintain normal blood sugar and allow release of glucose into the blood. The δ cells make up about 5 to 10% of pancreatic islet cells and secrete somatostatin which further regulates glucose levels. The ε cells and PP cells secrete ghrelin and pancreatic polypeptides, respectively. Pancreatic polypeptide-producing cells (about 5 to 10% of pancreatic islet cells) release hormones which alter exocrine and gastrointestinal functions. Pancreatic islet cells also include other types such as endothelial cells, neurons and progenitor cells.

The term "pancreatic islet cells" as used herein includes these pancreatic islet cells and pancreatic islet precursor cells which are progenitors of pancreatic islet cells, and they may also be intermediate cells that develop on the way to pancreatic islet cells or pancreatic islet precursor cells, produced during the course of development into pancreatic islet cells or during the course of inducing differentiation from somatic stem cells or pluripotent stem cells. The term "intermediate cells" preferably refers to cells whose destiny for differentiation to pancreatic islet cells has been determined. According to the invention, pancreatic islet cells may be created from pancreatic islet-like cells or pancreatic islet precursor-like cells in which the Mycl gene or its gene product has been introduced.

In type I diabetes, cellular infiltration (of primarily lymphocytes) into the pancreatic islets is seen soon after onset. This eventually leads to selective loss of β cells, and reduction in pancreatic islet volume, leaving primarily α cells. While insulin secretion by pancreatic islets is partially reserved, disappearance of β cells and lack of further secretion of the required amount of insulin results in onset of type I diabetes. In type II diabetes, on the other hand, most cases have no morphological changes such as notably reduced β-cell counts in the pancreatic islets. The major cause of type II diabetes is impaired regulation of blood glucose by insulin, resulting from insulin resistance in peripheral tissues.

According to the invention, the origin or source of pancreatic islet cells into which the Mycl gene or its gene product is transferred is not restricted and may be primary islet cells isolated from the pancreas of an individual or pancreatic islet cells cultured by a known culturing method. The cultured pancreatic islet cells are not limited, and may include stocked pancreatic islet cells, or pancreatic islet cells derived from stem cells (such as iPS cells, ES cells or somatic stem cells) (see Kimura, A., et al., Cell Chemical Biology, 2020, doi.org/10.1016/j.chembiol.2020.08.018, for example). The pancreatic islet cells into which the Mycl gene or its gene product is to be transferred according to the invention may also be pancreatic islet cells obtained from pancreatic islet-like cells and/or pancreatic islet precursor-like cells, and the Mycl gene or its gene product may also be again transferred into pancreatic islet-like cells and/or pancreatic islet precursor-like cells into which the Mycl gene has already been transferred. Both pancreatic islet cells harvested from a donor and pancreatic islet cells derived from stem cells are suitable for the purpose of treating diabetes. Pancreatic islet cells from a donor may be either autologous or heterologous with respect to the recipient. According to the invention, transfer of the Mycl gene into pancreatic islet cells to cause proliferation of pancreatic islet precursor-like cells *in vitro,* and returning the cells (grafting) into a patient, can be used for treatment of diabetes. Thus, in cases where the pancreatic islet cells are heterologous with respect to the patient, they may be used in combination with known methods such as appropriate administration of an immunosuppressive agent, matching of transplant donor tissue with the HLA form of the recipient, or embedding of donor tissue in alginic acid or an alginic acid derivative or semipermeable membrane. Grafting into the patient may be with pancreatic islet-like cells, pancreatic islet precursor-like cells, insulin-producing cells, or any desired combination thereof.

For loss of β cells, at least in mice, previous reports have been published in regard to proliferating α cells and differentiating a portion of the α cells into β cells. Pancreatic islets used as a source of pancreatic islet cells into which the Mycl gene is to be transferred may therefore be normal pancreatic islets containing large numbers of β cells, or pancreatic islets which have lost most of their β cells. From this viewpoint the present invention can also be applied for gene therapy of type I diabetes patients with loss of β cells, using pancreatic islet-like cells having the Mycl gene transferred.

### (3) Pluripotent stem cells

The term "pluripotent stem cells" used herein refers to cells with self-renewal capability and pluripotency, the cells having the ability to form different types of cells composing the body. The term "self-renewal capability" means the ability to produce identical undifferentiated cells from a single cell. The term "differentiation potency" means the ability of a cell to differentiate. Examples of pluripotent stem cells include embryonic stem cells (ES cells), induced pluripotent stem cells (iPS cells), Muse cells (Multi-lineage differentiating Stress Enduring cells), germline stem cells (GS cells) and embryonic germ cells (EG cells), with no limitation to these. The pluripotent stem cells used for the invention are preferably ES cells. Pluripotent stem cells may be from a mammal, bird, fish, reptile or amphibian, with no particular limitations. Mammals include primates (humans, monkeys, etc.), rodents (mice, rats, guinea pigs, etc.), cats, dogs, rabbits, sheep, pigs, cows, horses, donkeys, goats and ferrets.

The term "ES cells" used herein refers to pluripotent stem cells having the ability to differentiate into all tissue cells of the body during early development, which may be stocked so as to be taken and cultured *in vitro.* ES cells can be increased essentially indefinitely while retaining the ability to differentiate into all cells of the body similar to pluripotent stem cells of the early embryo. Mouse ES cells, specifically, were first described in 1981 (Proc. Natl. Acad. Sci. USA 78, 7634-7638, 1981; Nature 292, 154-156, 1981). ES cells are pluripotent and can generate all tissues and cell types of the body. Pluripotent embryonic stem cells have been isolated from numerous and various species including rat (Iannaconns et al., Dev. Biol. 163, 288-292, 1994), hamster (Dev. Biol. 127, 224-227, 1988), rabbit (Mol. Reprod. Dev. 36, 424-433, 1993), bird, fish and pig (Reprod. Fertil. Dev. 6, 563-568, 1994), cow (Reprod. Fertil. Dev. 6, 553-562, 1994) and primate (Proc. Natl. Acad. Sci. USA 92, 7844-7848, 1995). ES cells to be used for the invention are not restricted and may be KH2 cells, RF8 cells, JI cells, CGR8 cells, MG1.19 cells, 129SV cells, C57/BL6 cells or DBA-1 cells.

Several research teams have successfully isolated ES cells and ES cell-like stem cells from embryonic human tissue. The initial successes were the following: Science 282, 1145-1147, 1998; Proc. Natl. Acad. Sci. USA 95, 13726-13731, 1998; Nature Biotech., 18, 399-404, 2000. These ES cell lines were established by culturing ICM isolated from blastocysts on feeder cells. Other recent research indicates that nuclei from embryos and mature mammalian cells can be grafted into enucleated oocytes to obtain embryos and embryonic cells.

According to the invention it is possible to use any established ES cell line. Alternatively, it is effective to create a clone embryo using somatic cells from an individual and to establish an ES cell line from it, in order to prevent immunorejection that occurs when ES cells prepared by the method of the invention are used in an individual. Using this method allows ES cells to be established that have the same genetic elements as the individual.

Alternatively, the phenomenon of "reprogramming" may be used, whereby somatic cell nuclei introduced into eggs when creating somatic cell clones change into the same state as fertilized ovum nuclei. It has also been reported that ES cells have activity similar to this type of activity of eggs (Curr. Biol., 11, 1553-1558, 2001). In other words, fusion of individual somatic cells and ES cells is expected to allow somatic cells to be converted to cells such as ES cells. Since ES cells can be genetically engineered *in vitro,* carrying out the procedure using ES cells with prior manipulation of the factors associated with immunological rejection, such as the MHC gene group, makes it potentially possible to avoid rejection without using a method such as somatic cell clone embryo creation.

As used herein, the term "induced pluripotent stem (iPS) cells" refers to cells with pluripotent differentiating power similar to ES cells, obtained by introducing genes for transcription factors such as Oct3/4, Sox2, Klf4 and c-Myc into somatic cells. It is likewise possible to indefinitely increase iPS cells that retain pluripotent differentiating power, similar to ES cells.

One basic method for creating iPS cells involves using viruses for introduction of the four transcription factors Oct3/4, Sox2, Klf4 and c-Myc into cells (Takahashi K, Yamanaka S: Cell 126(4), 663-676, 2006; Takahashi, K, et al: Cell 131(5), 861-72, 2007). Examples of cells to be used for creating iPS cells, i.e. cells from which iPS cells can be derived, include lymphocytes (T cells and B cells), fibroblasts, epithelial cells, endothelial cells, mucosal epithelial cells, mesenchymal stem cells, hematopoietic stem cells, adipose stem cells, dental pulp stem cells and neural stem cells.

Reprogramming of iPS cells can be carried out by a method known to those skilled in the art, such as Addgene's Blog/Post, "Delivery Methods for Generating iPSCs" (https://blog.addgene.org/delivery-methods-for-generating-ipscs). The method for transferring the Mycl gene into iPS cells is not restricted and may be a method of introduction using a recombinant virus (such as retrovirus, lentivirus, adenovirus or Sendai virus), a recombinant plasmid, minicircle or episome (such as oriP/Epstein-Barr nuclear antigen-1 (EBNA1) episomal vector), or a method of introducing RNA (including mRNA) encoding the Mycl gene or the Mycl protein itself directly into cells.

The term "EG cells" as used herein refers to any embryonic germ cells created from primordial germ cells, with no particular restriction on their source. Also as used herein, "GS cells" refers to a cell line of germ line cells created from testes germ cells, which allow spermatogonial stem cells (germline stem cells) to be cultured *in vitro* (Cell. 119, 1001-1012, 2004). Preferred among GS cells are mGS cells (multipotent germline stem cells) that have pluripotent differentiating power and a nature similar to that of ES cells.

### (4) Mycl gene and its gene product

The Mycl (also "L-Myc") gene is a member of the Myc gene family which includes the c-Myc gene and Mycn ("N-Myc") gene. The Mycl gene is an oncogene similar to the c-Myc gene, and is known as a reprogramming gene. The Mycl gene also differs from the c-Myc gene in having virtually no transformation ability (Nakagawa, M., et al., Proc. Natl. Acad. Sci. USA, vol. 107, p.14152-14157, 2010). The cDNA sequence information for mouse and human Mycl can be obtained by referring to NCBI Accession No. NM_008506 and NM_001033081, respectively, and a person skilled in the art can easily isolate the cDNA.

According to the invention it is preferable to use the isolated Mycl gene and gene product. As mentioned above, the nucleotide sequence of the Mycl gene can be identified based on NCBI Accession No., and usable Mycl genes include single-stranded or double-stranded DNA, and their RNA complements. DNA includes naturally-derived DNA, recombinant DNA, chemically synthesized DNA, DNA amplified by PCR, chemically modified DNA, and combinations of the foregoing. DNA is preferred as the nucleic acid to be used for the invention. It is well known that the genetic code is degenerate, with some amino acids having multiple nucleotide sequences coding for the same amino acid, and there are no particular restrictions so long as the pancreatic islet cells into which the Mycl gene has been transferred (pancreatic islet-like cells) undergo cell proliferation by expression of the Mycl gene, and exhibit accelerated insulin production when its expression is stopped.

According to one embodiment, the Mycl gene may include:
(1) a nucleic acid which comprises or consists of the nucleotide sequence represented by SEQ ID NO: 1 or 3; or
(2) a nucleic acid encoding a polypeptide that hybridizes with nucleic acid comprising or consisting of the nucleotide sequence represented by SEQ ID NO: 1 or 3 under stringent conditions, and having a function of sustaining and/or promoting proliferative activity of pancreatic islet-like cells when expression of the Mycl gene has been induced; or
(3) a nucleic acid encoding a polypeptide that hybridizes with nucleic acid comprising or consisting of the nucleotide sequence represented by SEQ ID NO: 1 or 3 under stringent conditions, having an effect of causing proliferation of pancreatic islet-like cells in which the Mycl gene has been transferred, resulting in continuous proliferation of insulin-producing cells, and consequently an effect of continuously promoting insulin production.

The phrase "under stringent conditions" as used herein means hybridizing under moderately or highly stringent conditions. Specifically, moderately stringent conditions can be easily determined by a person skilled in the art with access to common technology, based on the DNA length. The basic conditions are described by Sambrook, J. et al. in Molecular Cloning, A Laboratory Manual (3rd edition), Cold Spring Harbor Laboratory, 7.42-7.45 (2001), but for a nitrocellulose filter, and a pre-rinsing solution of 5 × SSC, 0.5% SDS, 1.0 mM EDTA (pH 8.0), the conditions used may be hybridization conditions with ~50% formamide, 2 × SSC to 6 × SSC at about 40 to 50°C (or another similar hybridization solution such as Stark's solution in ~50% formamide at about 42°C), and rinsing conditions with 0.5 × SSC, 0.1% SDS at about 60°C. Highly stringent conditions can also be easily determined by a person skilled in the art based on the DNA length, for example. Such conditions are generally hybridization and/or rinsing with a higher temperature and/or lower salt concentration than moderately stringent conditions, and for example, such hybridization conditions are defined as rinsing with 0.2 × SSC, 0.1% SDS at about 68°C. A person skilled in the art will appreciate that the temperature and rinsing solution salt concentration can be adjusted as necessary depending on factors such as probe length.

Homologous nucleic acid cloned using nucleic acid amplification reaction or hybridization has 30% or greater, preferably 50% or greater, more preferably 70% or greater, even more preferably 90% or greater, yet more preferably 95% or greater and most preferably 98% or greater identity with the respective nucleotide sequences listed as SEQ ID NO: 1 or 3. The percent identity can be determined by visual examination and mathematical calculation. Alternatively, the percent identity of two nucleic acid sequences can be determined by comparing sequence information using the GAP computer program mentioned in Devereux et al., Nucl. Acids Res., 12, 387(1984) and available from University of Wisconsin, Genetic Computer Group (UWGCG) (GCG Wisconsin Package, version 10.3).

According to one embodiment, the gene product of the Mycl gene is the polypeptide expressed by the Mycl gene. Typically, the polypeptide may be:
(1) a polypeptide which comprises or consists of the amino acid sequence represented by SEQ ID NO: 2 or 4; or
(2) a polypeptide having at least 80%, 85%, 90%, 95%, 97%, 98% or 99% sequence identity with the amino acid sequence represented by SEQ ID NO: 2 or 4, and having the function of sustaining and/or promoting the proliferative activity of pancreatic islet-like cells in a glucose concentration-dependent manner.

According to one embodiment, the gene product of the Mycl gene may be a mutant polypeptide as defined above, and may be the amino acid sequence of SEQ ID NO: 2 or 4 having a deletion, substitution, insertion and/or addition of one or more amino acids. A substitution may be a conservative substitution, which is a substitution of specific amino acid residues with residues having similar physicochemical properties. Non-limitative examples of conservative substitutions include substitutions among aliphatic group-containing amino acid residues, such as mutual substitutions of Ile, Val, Leu and Ala, and substitutions among polar residues, such as mutual substitutions of Lys and Arg, Glu and Asp or Gln and Asn.

Mutations by deletion, substitution, insertion and/or addition of amino acids can be produced by the well-known technique of site-directed mutagenesis (Nucleic Acid Research, Vol. 10, No. 20, p.6487-6500, 1982, for example) of the Mycl gene. As used herein, the term "one or more amino acids" means a number of amino acids that can be deleted, substituted, inserted and/or added by a site-directed mutagenesis method. The term "one or more amino acids" used herein may also refer merely to one or several amino acids.

The method of deleting, substituting, inserting and/or adding one or more amino acids to the amino acid sequence of a polypeptide while retaining its activity may also be, instead of the aforementioned site-directed mutagenesis, a method of treating the gene with a mutation source, or a method of selectively cleaving the gene and then removing the selected nucleotide and then making a substitution, insertion or addition followed by linkage. Without being limitative, the gene product of the Mycl gene of the invention may be a polypeptide having an amino acid sequence with a deletion, substitution or addition of 1 to 10, preferably 9 or fewer, 7 or fewer, 5 or fewer, 3 or fewer or 2 or fewer amino acids, and more preferably 1 amino acid, in SEQ ID NO: 2 or 4, and having activity of promoting insulin production.

The mutant is a protein comprising an amino acid sequence having at least 80% or greater, preferably 85% or greater, 90% or greater, 95% or greater, 96% or greater, 97% or greater, 98% or greater or 99% or greater amino acid identity with the amino acid sequence listed as SEQ ID NO: 2, the polypeptide having an effect of causing proliferation of pancreatic islet-like cells and/or an effect of promoting insulin production.

The percent identity between two amino acid sequences can be determined by visual examination and mathematical calculation. Alternatively, the percent identity between two protein sequences can be determined by comparing sequence information using the GAP computer program available from University of Wisconsin, Genetic Computer Group (UWGCG), based on the algorithm described in Needleman, S.B. and Wunsch, C.D. (J. Mol. Biol., 48:443-453, 1970). The preferred default parameters for the GAP program include: (1) Scoring matrix, blosum62, as described in Henikoff, S. and Henikoff, J.G. (Proc. Natl. Acad. Sci. USA, 89:10915-10919, 1992), (2) 12 gap weight, (3) 4 gap weight; and (4) no penalty for end gap.

### (5) Transfer of Mycl gene

According to the invention, the method of introducing the Mycl gene into primary islet cells, cultured islet cells or stem cells (such as iPS cells, ES cells and somatic stem cells) is not particularly restricted, and any method publicly known to those skilled in the art may be used. Common gene transfer means are "transformation" and "transfection", which refer to transient or stable genetic changes induced in cells after incorporating exogenous nucleic acid (such as exogenous DNA or RNA for the host cells). Genetic changes can be obtained by incorporating exogenous nucleic acid into the genome of host cells, or by transiently or stably maintaining exogenous nucleic acid either as an episome component or independently. According to the invention, the introduced Mycl gene may be incorporated into the host cell genome so long as it is possible to control on/off switching of the gene expression, or it may be present as an episome component, or it may be present in the cytoplasm as a plasmid or vector containing the gene.

A "vector" is commonly used for introduction of exogenous nucleic acid (preferably DNA) into host cells. Vectors commonly include viruses, and particularly attenuated viruses and/or non-replicatable viruses. A viral vector may be a retrovirus vector, a lentivirus vector, an adenovirus vector, an adeno-associated virus vector or a Sendai virus vector. The vector may also include a regulatory sequence such as a promoter, enhancer, ribosome-binding sequence, terminator or polyadenylated site, to allow exogenous nucleic acid expression. If necessary, it may also include a selective marker sequence such as a drug resistance gene (a kanamycin resistance gene, ampicillin resistance gene or puromycin resistance gene, for example), a thymidine kinase gene or a diphtheria toxin gene, or a reporter gene sequence such as a fluorescent protein, β-glucuronidase (GUS) or FLAG. The term "promoter" used here is intended to include promoter components that are sufficient for cell type-specific controllable, tissue-specific controllable or exogenous signal- or chemical agent-inducible promoter-dependent gene expression. Such components may be located at the 5'-region or 3'-region of a natural gene. The term "functionally linked" means that a DNA sequence and regulatory sequence are linked in such a manner as to allow expression when a suitable molecule (such as a transcription activation protein) binds to the regulatory sequence.

Introduction of the vector into the host cells is not particularly restricted and may be carried out by the electroporation method (Meiner, V. et al., Proc. Natl. Acad. Sci. USA, 93:14041-14046(1996)), the calcium phosphate method, the DEAE-dextran method or a method using gene transfer lipids (lipofectamine or lipofectin). The vector-transferred cells may then be selected based on the property of a marker gene (such as a drug resistance gene). This can be confirmed by Southern blotting in which the probe used is a portion of exogenous nucleic acid that is the target of proper homologous recombination in the selected cells. It is thus possible to create cells containing a heterologous gene comprising the target gene, and specifically the Mycl gene, with knock-in of the marker gene.

The ES cells used may be the KH2 line having the Frt sequence downstream from the Cola1 gene locus, and expressing the reverse tetracycline regulatory transactivator M2-rtTA under the control of the endogenous Rosa26 promoter (Beard C, et al., Genesis, vol. 44, p.23-28(2006)). Introduction of the Mycl gene into the ES cells can be carried out using a method known to those skilled in the art, and for example, pCR8-GW-TOPO vector (Invitrogen Life Technologies) which is an entry vector, may be inserted by TA cloning of the Mycl gene, and then LR reaction may be carried out between this vector and the Colla1-TetOP-AttR1-ccdB-AttR2-ires-mCherry vector, for example, and the TetOP-Mycl-ires-mCherry vector used as a gene transfer vector. The "TetOP (operon)" sequence in the vector is a sequence to which the reverse tetracycline regulatory transactivator binds (tetracycline response element: TRE), and it binds with a reverse tetracycline regulatory transactivator expressed from host cells in a manner dependent on reverse tetracycline, such as doxycycline (Dox) added to the cells, inducing expression of the gene linked downstream from it. The "ires" (internal ribosome entry site) is a ribosome internal recognition sequence, while "mCherry" is a gene coding for red fluorescent protein (reporter gene). By introducing the vector into KH2-ES cells together with nucleic acid coding for flipase, it is possible to incorporate the Mycl gene into ES cell chromosomes. In addition to the vector including "ires-mCherry", a similar vector may be used which contains pBSSK(-)-IRES-βgeo comprising a "ires-βgeo cassette" containing resistance genes (fused β-galactosidase and neomycin resistance genes) (Mountford P. et al., Proc. Natl. Sci. USA, 91:4303-4307(1994), and IRES-Hygro (hygromycin resistance gene) cassette.

The present invention allows transient expression of the Mycl gene to be regulated to cause proliferation of pancreatic islet-like cells and differentiation into insulin-producing cells. The invention is thus characterized by transient expression of the Mycl gene, but when the Mycl gene is transiently expressed, the relative number of cells in which the Mycl gene has been transferred decreases as the pancreatic islet-like cells undergo cell division, and therefore expression level of the Mycl gene can naturally decrease or stop as time progresses. In other words, according to this embodiment the object of the invention is achieved by "on" regulation of Mycl gene expression. According to another embodiment, Mycl gene expression can be forcibly regulated to be "off" (also referred to hereunder as "on/off" regulation).

Using reverse tetracycline in this manner allows on/off regulation of expression of the Mycl gene that has been introduced into the host cells. In other words, intracellular expression of the Mycl gene can continue in the presence of reverse tetracycline, causing proliferation of pancreatic islet-like cells, while removing reverse tetracycline can halt proliferation of pancreatic islet-like cells and induce their differentiation to insulin-producing cells. According to the invention, the transient expression ("on" state) of the Mycl gene introduced into the pancreatic islet-like cells is preferably for a period of from at least 2 days to a maximum of 100 days, such as 90 days, 80 days, 70 days or 60 days, after the start of cell culturing. The pancreatic islet-like cells into which the Mycl gene has been introduced preferably proliferate during this period.

An alternate means of on/off regulation of Mycl gene expression is a "photoregulated viral vector" (Tahara, M., et al., PNAS, vol. 116, 11587-11589, 2019) that allows gene expression of a viral vector and cell proliferation to be precisely switched on and off by photoirradiation. Such a vector has a gene coding for an optical switch protein known as a magnet introduced into a viral vector, with expression of the target gene incorporated into the same vector being controlled using blue light.

Other alternative examples for on/off regulation of Mycl gene expression include an episomal vector (Okita K., et al., Nat Methods 2011 May 8(5):409-412), Sendai virus, or an RNA vector (Warren L., et al., Cell Stem Cell 2010 Nov 7(5):618-630). There is no limitation to these, and methods using reprogramming (establishment) of iPS cells (i.e. methods of temporarily inducing genes) may also be used.

According to the invention, the object of the invention can also be achieved by causing forced expression of the Mycl gene present in cells instead of introducing an isolated exogenous Mycl gene. The means for causing expression of an endogenous Mycl gene is not limited, and it may be a method of replacing a wild type promoter or enhancer with a strong promoter or enhancer for inducing operable expression of the Mycl gene, and forcibly causing gene expression. Examples of promoters to be used for such replacement include the strong promoters: cytomegalovirus (CMV) promoter, and inducible promoters that function in the presence of inducers. Examples of enhancers to be used for the replacement include SV40 enhancer, herpes B virus enhancer, cytomegalovirus enhancer and α-fetoprotein enhancer. According to another embodiment, a molecule may be used that has promoter- and/or enhancer-activating demethylating enzyme, histone-modifying enzyme or transcription activator, such as VP64, p65 or Rta, added to the genome-recognition sequence of CRISPR TypeII (including CRISPR-dCas9), or CRISPR-type I, TALEN or ZFN. Throughout the present specification, the promoter, enhancer and other activating enzymes and factors, or nucleic acid protein complexes or low molecular compounds, may be referred to as "activators" for activation of the Mycl gene.

### (6) Introduction of Mycl gene product

Introduction of Mycl gene product into cells may be carried out by a common method for introducing exogenous genes or proteins into cells. Such methods include, but are not limited to, methods using transfection reagents, methods using viruses, electroporation methods, particle gun methods, sonoporation methods, liposome fusion methods and transfer methods in which pores are formed in cell membranes using a micromanipulator or laser light irradiation.

### (7) Creation of chimeric mammals

The method of introducing ES cells into a mammal to create a chimeric mammal may be a method that is well known to those skilled in the art. First, any medium known to those skilled in the art may be used for culturing of ES cells into which the Mycl gene has been introduced. For example, when the ES cells are to be cultured on feeder cells, the feeder cells used may be MEF (mouse embryonic fibroblasts), and the ES cells may be cultured on the feeder cells using ES cell medium (for example, knockout DMEM (GIBCO) containing 15% FBS, 50 U/mL penicillin/streptomycin, L-glutamine and non-essential amino acids, with addition of 2-mercaptoethanol (2ME, GIBCO) and LIF (SIGMA).

The ES cells are then introduced into a mammal to create a knockout animal (Mycl gene knock-in animal). Mice were used here as an example of mammals, and methods of creating knock-in mice are well known to those skilled in the art. Specifically, the ES cells may be injected into a mouse (such as C57BL/6) blastocyst, which is then transplanted into the uterus of a pseudopregnant female mouse (such as ICR) to create a chimeric mouse. The chimeric mouse may then be cross-bred with a normal mouse such as C57BL/6) to create a hetero mutant mouse with hetero knock-in of the Mycl gene. Cross-breeding of hetero mutant mice can produce a homozygous mouse with homo knock-in of the Mycl gene. Creation of the knock-in mice may be the same as for ECAT3 knock-in mice (Tokuzawa, Y., et al., Molecular and Cellular Biology, 23(8):2699-2708(2003)), ECAT4 knock-in mice (Mitsui, K., et al., Cell, 113:631-642(2003)) or ECAT5 knock-in mice (Takahashi, K., K. Mitsui and S. Yamanaka, Nature, 423(6939):p541-545(2003), Japanese Unexamined Patent Publication No. 2003-265166).

Chimeric mammals can also be created using iPS cells instead of the aforementioned ES cells. For example, a blastocyst complementation method may be used to create an organ from human iPS cells in a non-human mammal. For example, Nakauchi et al. have generated human pancreas derived from human iPS (Nakauchi, H., et al., PNAS, Vol. 110, No. 1, 4557-4562(2013)) in the bodies of pancreas-lacking cloned pigs.

### 2. Regulation of proliferation and differentiation of pancreatic islet-like cells

According to the invention it is possible to control proliferation and differentiation of pancreatic islet-like cells created as described above, both *in vitro* and *in vivo.* The method of inducing the Mycl gene can be selected according to the method used for introduction of the gene into cells. For example, when the TetOP-Mycl-ires-mCherry vector has been used as the gene transfer vector for introduction of Mycl gene into cells, it binds with reverse tetracycline regulatory transactivator expressed by host cells in a manner dependent on a reverse tetracycline agent such as doxycycline (Dox), and the Mycl gene linked downstream from it is expressed, to allow proliferation of pancreatic islet-like cells to be induced. The concentration of Dox added to the cell culture system may be adjusted as appropriate. It may be 1 to 100 mg/mL, for example. After pancreatic islet-like cells have proliferated by addition of Dox, the proliferation can be stopped by replacement with Dox-free medium, for example, to induce cell differentiation into insulin-producing cells.

Addition of Dox-containing water to a chimeric non-human mammal *in vivo* can induce proliferation of pancreatic islet-like cells in the pancreas. The Dox concentration when water has been added may be 1 to 100 mg/mL and preferably 2.0 mg/mL, for example. When a chimeric non-mouse is used, since the pancreas grows with increasing age up to 8 weeks after birth, Dox may be administered during the period when growth has ceased, such as after 8 weeks from birth, but there is no limitation to the number of weeks so long as proliferation can be induced.

### 3. Method for producing pancreatic islet cells or progenitor cells with insulin-producing ability

The invention provides a method for producing pancreatic islet cells or their progenitor cells which have insulin-producing ability. The production method is not restricted but includes using primary islet cells, cultured islet cells or stem cell-derived pancreatic islet cells as the source, introducing the Mycl gene into the pancreatic islet cells, and causing proliferation of the cells by forced expression of the Mycl gene, and then stopping expression of the gene to obtain pancreatic islet cells or progenitor cells with insulin-producing ability. As explained above, forced expression and stopping expression of the Mycl gene may utilize alternative means for on/off regulation of the gene expression (such as using a photoregulated viral vector), or a doxycycline-sensitive reverse tetracycline regulatory transactivator. The term "pancreatic islet progenitor cells" or "pancreatic islet progenitor cells" used herein refers to cells (or a cell group) on the pathway of differentiation to pancreatic islet cells with insulin production ability after expression of the Mycl gene has been stopped, and they can be identified using one or more markers selected from the group consisting of PDX1 positivity, PTF1a positivity, NKX6.1 positivity, Fev positivity, Pax4 positivity, and the Cck gene.

### 4. Method of proliferating pancreatic islet-like cells

With the prior art there has been a limit to the amplification efficiency for mature pancreatic islet cells by transfer of the Mycl gene. In particular, it was found that the amplification efficiency is lower with more prolonged proliferation of pancreatic islet-like cells. According to the invention it is possible to induce further proliferation of mature pancreatic islet cells by the Mycl gene, by applying stimulation with higher-concentration glucose. The invention therefore provides a method of culturing pancreatic islet cells, in which the Mycl gene or its gene product has been transferred or where its expression has been induced, using medium containing high-concentration glucose, i.e. a method of sustaining and/or promoting proliferation activity for pancreatic islet cells in which the Mycl gene or its gene product has been transferred or where its expression has been induced, in a glucose concentration-dependent manner. The phrase "glucose concentration-dependent manner" means that stimulation with higher-concentration glucose can induce further proliferation of pancreatic islet cells in a fixed concentration range (for example, 10 g/L or lower). According to one embodiment, culturing of pancreatic islet-like cells is carried out in medium containing high-concentration glucose (also referred to simply as "high-glucose medium"), matching transient expression ("on" state) of the Mycl gene. High-glucose medium is medium having a glucose concentration of 3 g/L or greater, and it is preferably medium with a glucose concentration of 3.5 to 10 g/L, more preferably 4 to 7 g/L and even more preferably 4 to 6 g/L.

According to one aspect of using high-glucose medium, culturing may be carried out for a fixed period of time with high-glucose medium and followed by culturing for a fixed period of time with low-glucose medium, and then culturing may again be carried out for a fixed period of time with high-glucose medium, thus alternately applying (using) the high-glucose medium and low-glucose medium. By alternately applying (using) high-glucose medium and low-glucose medium for culturing, it is possible to maintain the proliferative activity of pancreatic islet-like cells for prolonged periods. The term "low-glucose medium" is used interchangeably with "low-concentration glucose medium", and refers specifically to medium having a glucose concentration of lower than 3 g/L, and preferably medium having a glucose concentration of 0.1 to 2.8 g/L, more preferably 0.5 to 2.5 g/L and even more preferably 1 to 2 g/L. The timing for exchange between the high-glucose medium and low-glucose medium may be adjusted as appropriate, and for example, it may be adjusted to the timing of subculturing of the pancreatic islet-like cells (such as, without being limitative, once per day, once every 2 days, once every 3 days, once every 4 days, once every 5 days, once every 6 days or once every 7 days). By changing the glucose concentration it is possible to maintain proliferative activity of pancreatic islet-like cells for prolonged periods of, for example, one month or longer, 3 months or longer, 6 months or longer, 8 months or longer or 1 year or longer. From the viewpoint of suitably maintaining the function of maturation from pancreatic islet-like cells to pancreatic islet cells, such as glucose-dependent insulin secretion ability, the period of culturing by changing the glucose concentration is less than 1 year, preferably less than 8 months, more preferably less than 6 months, even more preferably less than 3 months and yet more preferably less than 1 month.

By regulating the glucose-activated pathway, without being limited to glucose itself, it is possible to cause proliferation of pancreatic islet cells in which the Mycl gene or its gene product has been transferred or its expression has been induced. Regulation of the glucose-activated pathway, for the purpose of the present specification, includes a method of activating the glucose-stimulated activation pathway and a method of alternately repeating a period with activation of the glucose-stimulated activation pathway and a period without such activation. A glucose-stimulated activation pathway is a signal transduction pathway that is stimulated by glucose, and one example is the GK pathway. According to the invention, the substance that activates the glucose-stimulated activation pathway, which is added to sustain and/or promote proliferative activity of pancreatic islet-like cells by Mycl pathway stimulation in a concentration-dependent manner, is not particularly restricted so long as it activates a glucokinase pathway, and examples include, in addition to glucose, sucrose, glucokinase activator (the known Cpd A (Merck Millipore, CAS 603108-44-7; Sigma-Aldrich, 346021)), glucokinase activator II (Sigma-Aldrich, 500487) and glucokinase activator III (Merck Millipore, CAS 300353-13-3; Sigma-Aldrich, 509665).

High-glucose medium and low-glucose medium can be prepared by appropriately combining commercially available basal media for cell culturing. Basal media for cell culturing include primarily carbon sources and nitrogen sources, as well as trace nutrients such as amino acids (such as essential amino acids), and vitamins (such as water-soluble vitamins and fat-soluble vitamins), inorganic salts, proteins (such as albumin, transferrin and insulin), reducing agents (such as glutathione), trace elements (such as ammonium metavanadate, manganese chloride and sodium selenite), and other substances (such as ethanolamine, hypoxanthine sodium, fatty acids, putrescine, pyruvic acid, thymidine and phenol red), with the components and concentrations of the components being adjusted as appropriate. The basal medium to be used for preparation of the medium may be appropriately selected from among commercially available basal media, as media containing glucose in the aforementioned concentration, based on the component list published by each manufacturer. A specific component (especially an activator of a glucose stimulation-activated pathway) may be separately prepared and added to a preferred concentration, or a customized medium may be purchased from a culture medium manufacturer. Examples of basal media to be used include commercially available DMEM-F12, DMEM and RPMI 1640.

The basal medium may further include growth factors. Preferred growth factors to be added include, but are not limited to, EGF, FGF-10, Gastrin, Noggin, R-spondin and B27 supplements. For example, while it is generally known that FGF-10 has an effect of causing proliferation of cells (including pancreatic islet progenitor cells), the proliferative activity of pancreatic islet-like cells can be maintained by adjusting the glucose-activated pathway, with or without its addition. Throughout the present specification, therefore, the method of culturing by causing proliferation of pancreatic islet cells using an activator of a glucose stimulation-activated pathway does not need to include addition of FGF10, although FGF10 is preferably added from the viewpoint of causing more stable growth.

It is also possible to cause *in vivo* amplification of pancreatic islet cells into which the Mycl gene or its gene product has been transferred, or in which its expression has been induced. Specifically, the glucose-activated pathway may be adjusted *in vivo,* and one aspect may be administration to the body in the form of a buffering solution with the glucose concentration already adjusted to the final concentration, instead of "high-glucose medium" or "low-glucose medium". In this case, the term "high-glucose medium" may be considered to be "high-glucose buffer", and the term "low-glucose medium" may be considered to be "low-glucose buffer". The timing for exchange of the "high-glucose buffer" and "low-glucose buffer" is not restricted, and for example, it may be once per day, once every 2 days, once every 3 days, once every 4 days, once every 5 days, once every 6 days or once every 7 days. When ingested in a living body, it may be in the form of a beverage containing glucose or sucrose, which can be expected to sufficiently raise blood glucose.

### 5. Use as a drug

According to the invention it is possible to control expression of the transferred Mycl gene and regulate the glucose-activated pathway, thereby increasing proliferative activity for pancreatic islet-like cells, maintaining proliferative activity for pancreatic islet-like cells for prolonged periods, and promoting insulin production while also promoting proliferation of pancreatic islet-like cells. According to one aspect, therefore, there is provided an insulin production promoter that comprises, as an active ingredient, pancreatic islet-like cells that have their proliferative activity sustained or promoted for long periods with the Mycl gene or its gene product and an activator of a glucose stimulation-activated pathway such as glucose; a pharmaceutical composition comprising the aforementioned active ingredient and other pharmaceutically acceptable components (for example, a carrier, excipient, disintegrator, buffering agent, emulsifying agent, suspending agent, soothing agent, stabilizer, preservative, antiseptic agent or physiological saline); a method for preventing and/or treating a diabetes patient using the insulin production promoter or pharmaceutical composition; and the use of the Mycl gene and the activator of a glucose stimulation-activated pathway for production of the insulin production promoter or pharmaceutical composition.

Another aspect of the invention provides a method (*ex-vivo* method) for controlling expression of a transferred Mycl gene and regulating the glucose-activated pathway, in order to increase proliferative activity, and transplanting into a subject pancreatic islet-like cells wherein the proliferative activity of the pancreatic islet-like cells is sustained and promoted for a prolonged period, for the purpose of preventing and/or treating diabetes. The *ex-vivo* method of the invention may be intended for a different aspect of the invention, depending on the cells used. Examples include (i) the aspect of heterologous *in vitro* or autologous *in vitro* production of pancreatic islet-like cells obtained by gene transfer into cells derived from adult pancreatic islets (the *ex-vivo* method including both in the narrow sense); (ii) the aspect of heterologous *in vitro* or autologous *in vitro* production of pancreatic islet-like cells obtained by introduction of the Mycl gene of pancreatic islet-like cells obtained by inducing differentiation, i.e. pancreatic islet cells derived from stem cells (iPS cells, ES cells, somatic stem cells, etc.) (the *ex-vivo* method in the wide sense); (iii) the aspect of proliferating pancreatic islet precursor-like cells obtained by functional expression of the Mycl gene in (i) and (ii) above; and (iv) the aspect of insulin-producing cells or pancreatic islet cells (without the Mycl gene) produced by differentiation from (i) to (iii) above.

According to one aspect of the invention, the cells may be either autologous or heterologous with respect to the patient and administered by a publicly known method, and specifically by using the pancreatic islets to be grafted in encapsulated form, by the method described in Nature Medicine volume 22, pp.306-311(2016)., doi:10.1038/nm.4030, Nature Biomedical Engineering volume 2, pp.810-821(2018)., DOI:10.1038/s41551-018-0275-1, EBioMedicine 12 (2016) 255-262., DOI:https://doi.org/10.1016/j.ebiom.2016.08.034. Encapsulation of the pancreatic islets to be grafted makes it possible to avoid the use of immunosuppressive agents or to reduce the dosage of immunosuppressive agents.

According to one aspect, isolated pancreatic islets may be gene-edited as appropriate. There are no particular restrictions on the gene to be edited, and specifically, preferred characteristics may be added during treatment, such as by repair of a mutant gene in the pancreatic islet cells by genome editing, modification of immunoreaction-inducing molecules including surface antigens such as HLA or GAD protein, or induction of immunological tolerance by deletion of beta-2 microglobulin or the RFX5, RFXANK, RFXAP or CIITA genes. The method of gene editing is not particularly restricted, and for example, it may employ a transposon vector such as piggyBAC, for expression of CRISPR-Cas9, CRISPR-Cas12, TALEN, ZFN, CRISPR-Cas3, CRISPR-TypeI-D, MAD7 or their modified forms, or functional molecules.

### (1) Indications

Target diseases for application of this aspect are diseases in which insulin is not sufficiently functioning in a living body. Such diseases typically include conditions in which low secretion is observed or type I diabetes, insulin-resistant conditions with relative insulin deficiency or type II diabetes, and type 1.5 diabetes, which is a condition intermediate between type I diabetes and type II diabetes. According to the invention, expression of the transferred Mycl gene is controlled to regulate a pathway activated by glucose, whereby the proliferative activity of pancreatic islet-like cells can be sustained and promoted for prolonged periods to promote insulin production, and an effect of lowering blood glucose in diabetes patients, for example, is exhibited. The disease to be targeted will typically be diabetes, but more specifically it is a disease, disorder or symptoms associated with severe hypoglycemia, type I diabetes (including latent progressive type-1 diabetes or type 1.5 diabetes), type II diabetes, impaired glucose tolerance, hyperglycemia, heterolipidemia, obesity or metabolic syndrome, or another specific mechanism or disease, examples of which include genetic abnormalities associated with pancreatic β cell function, genetic abnormalities associated with insulin function transfer mechanisms, or other diseases or conditions including pancreatic exocrine diseases, endocrine diseases, liver disease, drug agent or chemical substance-induced disease, infectious disease or rare immune mechanism-related conditions, as well as gestational diabetes. Diabetes complications resulting from diabetes (such as diabetic retinopathy and diabetic neuropathy) may also be target diseases. Insulin hyposecretion resulting from pancreas removal or partial pancreas excision performed due to pancreatitis or pancreatic cancer, may also be a target disease.

There is no particular restriction on the type of diabetes to be treated by the method described herein, but the method can provide treatment wherein physiological insulin is secreted in a manner dependent on blood glucose level, and is unlikely to cause hypoglycemia. When treating severe hypoglycemia, for example, since it is known to those skilled in the art that donor pancreatic islets exhibit a marked effect against severe hypoglycemia, a method described in the published literature may be used as one aspect. The method described in the published literature may be, but is not restricted to, the specific methods described in Diabetes Care 2016 Jul; 39(7):1230-1240., DOI: 10.2337/dc15-1988, The New England Journal of Medicine. 343(4):230-238., DOI: 10.1056/NEJM20,0007273430401, and The New England Journal of Medicine. 355(13):1318-1330., DOI:10.1056/NEJMoa061267.

For use in treatment of type I diabetes, one aspect for treatment of type I diabetes may be, but is not restricted to, optionally adjusting the number of pancreatic islet cells, pancreatic islet-like cells, pancreatic islet precursor-like cells or insulin-producing cells according to the insulin level, blood glucose level and/or C-peptide level, either constantly or during fasting, after glycemic load and/or after glucagon stimulation in a type I diabetes patient, and determining the dose of the agent for *in vivo* treatment by the method described herein. Considering the high risk of hypoglycemia for insulin-depleted type I diabetes patients, and the desirability of treatment using the method described herein, a type I diabetes patient with a C-peptide level of 0.5 ng/mL or lower, preferably 0.2 ng/mL or lower and more preferably 0.1 ng/mL or lower may be treated during fasting and/or during glucagon stimulation. A specific dose for the agent may be adjusted in light of transplant amounts of donor pancreatic islets that are commonly grafted to treat severe hypoglycemia. Specifically, the pancreatic islet cells, pancreatic islet-like cells, pancreatic islet precursor-like cells or insulin-producing cells may be in an equivalent of 500 IEQ/kg or greater, preferably 1000 IEQ/kg or greater, more preferably 2000 IEQ/kg or greater and even more preferably 5000 IEQ/kg or greater.

For treatment of type II diabetes, one aspect may be, but is not limited to, use as a treatment agent for insulin-dependent type II diabetes having an insufficient amount of insulin secreted in the body. Specifically, this may be optionally adjusting the number of pancreatic islet cells, pancreatic islet-like cells, pancreatic islet precursor-like cells or insulin-producing cells according to the insulin level, blood glucose level and/or C-peptide level, either constantly or during fasting, after glycemic load and/or after glucagon stimulation in a type II diabetes patient, and determining the dose of the agent for *in vivo* treatment by the method described herein. Considering that a desirable effect can be obtained for pathologies with insufficient insulin, a type II diabetes patient with a C-peptide level of 0.5 ng/mL or lower, preferably 0.2 ng/mL or lower and more preferably 0.1 ng/mL or lower may be treated during fasting and/or during glucagon stimulation. A specific dose for the agent may be adjusted in light of transplant amounts of donor pancreatic islets that are commonly grafted to treat severe hypoglycemia. Specifically, the pancreatic islet cells, pancreatic islet-like cells, pancreatic islet precursor-like cells or insulin-producing cells may be in an equivalent of 500 IEQ/kg or greater, preferably 1000 IEQ/kg or greater, more preferably 2000 IEQ/kg or greater and even more preferably 5000 IEQ/kg or greater.

For use as a treatment agent for latent progressive type I diabetes, it may be, but is not limited to, adjusting the amount of agent according to the amount of blood glucose level and/or C-peptide, in the same manner as for type I diabetes and or type II diabetes. In this case, the pancreatic islet cells, pancreatic islet-like cells, pancreatic islet precursor-like cells or insulin-producing cells caused to proliferate by the method described herein are preferably derived from the patient. For example, as one preferred aspect for treatment of latent progressive type I diabetes, if the autoantibody for pancreatic islet cells or the HLA type in blood can be examined beforehand and autologous pancreatic islet cells can be increased by the method described herein either before or after an insulin-dependent condition and/or insulin-depleted condition has been established, then treatment will not require an immunosuppressive agent, and progression to an insulin-dependent condition and/or an insulin-depleted condition can be prevented, or the insulin-dependent condition and/or insulin-depleted condition can be treated. In order to identify whether the condition is latent progressive type I diabetes, if an autoantibody for pancreatic islet cells or the HLA type in blood is contributing to latent progressive type I diabetes, then a publicly known method may be used, such as, though not limited to, indicating whether or not the patient is positive for one or more antibodies from among pancreatic islet-related autoantibody such as pancreatic islet cell antibody (ICA), GAD antibody, insulin autoantibody (IAA) or IA-2 antibody. It may also be confirmed, by a known method, whether or not HLA associated with latent progressive type I diabetes, such as HLA-DR4-DQA1*0301-B1*0401, is retained.

Considering that a therapeutic effect is exhibited by donor pancreatic islets, treatment of severe hypoglycemia is preferably by an *ex-vivo* method, but pancreatic islet cells may also be increased in the body (*in vivo*) by the method described herein. Since increased pancreatic islet cells have the same effect as an *ex-vivo* method, a treatment method by increasing pancreatic islets *in vivo* may likewise be suitably selected as a treatment method for severe hypoglycemia, similar to an *ex-vivo* method. For treatment of type I diabetes, type II diabetes and latent progressive type I diabetes as well, a method of increasing pancreatic islet cells in the body (*in vivo*) may be selected, similar to an *ex-vivo* method, in consideration of the gender, age, body weight, affected state of the patient, and state of the cells used. A method of increasing pancreatic islet cells in the body (*in vivo*) may also be combined with an *ex-vivo* method.

According to the invention it is possible to prevent and/or treat such diseases. The term "prevent", as used herein, means stopping or delaying onset/development or lowering the risk of onset/development of the disease or its symptoms. The term "treatment" includes reduction (alleviation) of characteristic symptoms or associated symptoms of a target disease, and prevention or retardation of aggravation of symptoms. The term "prevention" means halting or delaying onset or expression of a disease (disorder) or its symptoms, or reducing the risk of its onset or expression. The term "improvement", on the other hand, refers to alleviation (reduction), reversal, remission or curing (including partial healing) of a disease (disorder) or its symptoms.

### (2) Insulin production promoter and pharmaceutical composition

An insulin production promoter or pharmaceutical composition comprising, as an active ingredient, pancreatic islet-like cells with prolonged sustained or promoted proliferative activity provided by the Mycl gene or its gene product and the glucose stimulation-activated pathway activator of the invention, can be provided for prevention and treatment of the aforementioned target diseases. One aspect of the pancreatic islet cell proliferation promoter is an agent that causes proliferation of pancreatic islet cells of any one type, and preferably any two or more types, from among α cells, β cells, δ cells, ε cells and PP cells in pancreatic islets. Another aspect of the pancreatic islet function improver is an agent that improves some or all of the functions carried out by pancreatic islets in the body when it is administered, where some of the functions of pancreatic islets include, specifically, a blood sugar regulating effect by pancreatic islet cells, a hypoglycemic effect by insulin, a glucose-producing/releasing effect by glucagon, secretion-inhibiting effects on gastrin, secretin, insulin and/or glucagon by glucagon or a gastrointestinal tract nutrient absorption-inhibiting effect by somatostatin, an appetite-regulating effect by ghrelin, and gallbladder-contraction regulating effects and appetite-regulating effects by pancreatic polypeptides. Another aspect of the insulin production promoter is an agent that promotes physiological insulin secretion in response to blood glucose levels, as one of the functions performed by pancreatic islets in the body. When provided as a pharmaceutical composition, it may comprise other pharmaceutically acceptable components (for example, carriers, excipients, disintegrators, buffering agents, emulsifying agents, suspending agents, soothing agents, stabilizers, preservatives, antiseptic agents, physiological saline and the like), in addition to the active ingredient which is the Mycl gene or its gene product used according to the aspects described above. If necessary, the composition may also include an activator for activation of the Mycl gene.

Pancreatic islet cells derived from the body in which the Mycl gene or its gene product has been introduced may be pancreatic islet cells of a healthy individual, or pancreatic islet cells from a type I diabetes patient with loss of some or most β cells, or a terminal type II diabetes patient. The pancreatic islet cells may be either autologous or heterologous with respect to the recipient.

Without being limitative, the insulin production promoter and pharmaceutical composition of the invention may be obtained by suspending the pancreatic islet-like cells in physiological saline or an appropriate buffer solution (for example, phosphate-buffered saline). The number of cells necessary for treatment can be produced by causing forced expression of the Mycl gene for appropriate proliferation of the cells.

For use of the insulin production promoter and pharmaceutical composition, dimethyl sulfoxide (DMSO) or serum albumin may be added to the insulin production promoter or pharmaceutical composition to protect the cells, and an antibiotic may be added to prevent infiltration and growth of bacteria. In addition, other pharmaceutically acceptable components (for example, carriers, excipients, disintegrators, buffering agents, emulsifying agents, suspending agents, soothing agents, stabilizers, preservatives, antiseptic agents, physiological saline and the like) may be added to the insulin production promoter or pharmaceutical composition. A person skilled in the art may add such factors and chemical agents to the insulin production promoter and pharmaceutical composition in appropriate concentrations.

The number of pancreatic islet cells, pancreatic islet-like cells, pancreatic islet precursor-like cells or insulin-producing cells in the insulin production promoter and pharmaceutical composition to be prepared may be appropriately adjusted so as to obtain the desired effect for prevention and/or treatment of diabetes or its associated condition (for example, lowered blood glucose level), in consideration of the target gender, age and body weight, the state of the affected area, and the state of the cells to be used.

The insulin production-promoting agent and pharmaceutical composition of the invention may be administered to any of a variety of targets, including mammals such as primates, humans, dogs, cats, cows, horses, pigs or sheep, and preferably humans. The route of administration to the target is not restricted, and administration may be at any location that can produce a response to glucose in the body, by parenteral administration, such as injection or infusion, for example. Specifically, grafting or administration may be in the pancreas, under the renal capsule, preferably subcutaneous or intraperitoneal, more preferably intravascular or intravenous, and even more preferably intraportal.

The method of activating the administered Mycl gene in the body is not restricted, and a system that regulates "on" expression, or regulates "on and/or off" expression, of the Mycl gene may be used. For example, the "photoregulated viral vector" (Tahara, M., et al., PNAS, vol. 116, 11587-11589, 2019) mentioned above may be used.

For targeting of the Mycl gene into the pancreas, gene transfer may be carried out with a marker that is specifically expressed in pancreatic islet cells (for example, PDX1, C-peptide, insulin, MafA, Mnx1, Pax4, Pax6, NeruroD1, Isl1, Nkx2.2, Ngn3, HNF1a, Foxa2, Nkx6.1, glucagon, Arx, MafB, RFX6, IRX1, IRX2 and/or somatostatin) as the target. When a method of increasing pancreatic islet cells in the body (*in vivo*) is selected, the route of administration to the target is not restricted and may be, specifically, into the pancreas, subcutaneous or intraperitoneal, preferably intravascular or intravenous, and even more preferably into the celiac artery or into the pancreatic ducts.

### (3) Treatment method

According to the invention there is provided a method for preventing and/or treating a subject with diabetes or its associated condition, using pancreatic islet-like cells with proliferative activity that has been sustained or promoted for a prolonged period by the Mycl gene or its gene product and an activator of a glucose stimulation-activated pathway. Moreover, in the treatment method of the invention it is possible to administer an activator that can activate the Mycl gene, either before or simultaneously with administration of the insulin production promoter or pharmaceutical composition. In order to cause *in vivo* amplification of pancreatic islet cells into which the Mycl gene or its gene product has been transferred or in which its expression has been induced, the concentration of glucose in the body may be adjusted using diet, drinking water or drip infusion.

### (4) Kit

The present invention also provides a kit to be used for preventing and/or treating diabetes or its associated condition, which includes an insulin production promoter or pharmaceutical composition. Such a kit may also include an instruction manual for administration or grafting of the insulin production promoter or pharmaceutical composition. The kit may further include an activator for activation of the Mycl gene.

### EXAMPLES

The present invention will now be explained in more specific detail through the following Examples, with the understanding that the invention is in no way limited by the Examples.

### Method

### (i) Establishing ES cells capable of inducing Mycl expression in a doxycycline (Dox)-dependent manner

The ES cells used for transfer of the Mycl gene were of a KH2 line having the Frt sequence downstream from the Cola1 gene locus, and expressing the reverse tetracycline regulatory transactivator M2-rtTA under the control of the endogenous Rosa26 promoter (Beard C, et al., Genesis, vol. 44, p.23-28(2006)). Cloning of Mycl cDNA was carried out from the ES cell-derived cDNA, and the cloned fragment was inserted into pCR8-GW-TOPO vector (Invitrogen). A Col1a1-TetOP-Mycl-ires-mCherry vector prepared by LR reaction between the Mycl gene-inserted pCR8-Mycl-TOPO vector and TetOP-AttR1-ccdB-AttR2-ires-mCherry vector (hereunder referred to as "targeting vector") was inserted into the KH2-ES cell Col1a1 gene locus using a flip-in recombination system (Beard et al., 2006). For flip-in recombination, a cell suspension suspended in high-glucose DMEM (product of Nacalai Tesque) containing 50 µg of targeting vector in which the Mycl gene had been inserted, 25 µg of pFlapase vector and 25 mM HEPES buffer (Gibco), was electroporated into KH2-ES cells using a Gene pulser Xcell electroporation system (BIO-RAD) (two-pulse supply under conditions of Voltage: 550 V, Capacitance: 25 µF, Resistance: ∞, Cuvette: ≥4 mm). At 24 hours after electroporation, the cells were screened with 150 µg/mL of hygromycin B (Roche), and the formed colonies were collected and an ES cell line was established which was capable of inducing expression of the Mycl gene in Dox-dependent manner.

### (ii) Cell culturing method

Culturing of feeder cells (MEF; mouse embryo fibroblasts) was carried out using DMEM (Nacalai Tesque) containing 10% FBS (Gibco), 50 U/mL Penicillin-Streptomycin (P/S; Nacalai Tesque), L-glutamine (Gibco) and NEAA (Nacalai Tesque).

For culturing of the ES cells, medium obtained by adding 2-mercaptoethanol (2ME: Gibco) and LIF (Sigma) to knock out-DMEM (Gibco) containing 15% FBS, 50 U/mL P/S, L-glutamine and NEAA was used, with culturing carried out in a gelatin-coated (Sigma) feeder cell-seeded dish. For subculturing of the ES cells, treatment was carried out for about 3 minutes at 37°C with 0.25% trypsin/1 mM EDTA (Gibco), and an approximately 1/10 amount of cell suspension was seeded in a new dish.

### (iii) Creation of mice capable of Mycl expression induction in vivo (KH2-Mycl)

ES cells capable of Mycl expression induction in a Dox (Sigma-Aldrich)-dependent manner were injected into a mouse blastocyst (ICR, E3.5) and grafted into mice uterus on the 2nd day of pseudogestation (Slc:ICR, Shimizu Experimental Materials), to create chimeric mice having cells capable of Dox-dependent Mycl expression induction. The mice can be induced to systemically express the Mycl gene and red fluorescent protein mCherry by administration of Dox.

### (iv) Mice that allow tracking of pancreatic islet cells (Ins1-ires-CreERT2; Gcg-CreERT2, Sst-ires-CreERT2)

Mice were created having tamoxifen-inducible Cre recombinase (CreERT2) introduced downstream from the endogenous promoter of the Gcg gene specifically expressed by α cells, and downstream from endogenous genes, namely gene Ins1 specifically expressed by β cells and gene Sst specifically expressed by δ cells.

### (v) Doxycycline administration

Eight-week-old mice were used and were given a solution containing 2.0 mg/mL Dox as drinking water. Cultured cells were added to medium to a final concentration of 2.0 µg/mL.

### (vi) Preparation of pathological specimens of mouse organs

After dissecting the mice, each organ was transferred to 70% EtOH (obtained by diluting 100% EtOH by Wako Pure Chemical Industries, Ltd.) on the day following one day of shaking in 4% PFA (Wako Pure Chemical Industries, Ltd.), and was further shaken for one more day. On the following day, an STR120 spin tissue processor (Thermo Scientific) was used to fabricate a block according to the recommended protocol. The pathological specimens were prepared by request to BioGate Co., Ltd.

### (vii) Immunostaining

Each tissue section was immersed for 30 minutes or longer in xylene (Wako Pure Chemical Industries, Ltd.), and then in 100% EtOH (Wako Pure Chemical Industries, Ltd.). After washing with tap water for about 10 minutes, it was transferred into boiled antigen-activating solution at pH 9 (Nichirei Biosciences, 10-fold dilution), and subjected to antigen activating treatment for 10 minutes. Primary antibody solution diluted to different degrees with a blocking solution (2% BSA + 1 × PBS) was added at 200 µL onto the tissue section, and the mixture was allowed to stand for 30 minutes to 1 hour. After washing twice with 1 × PBS, 2 drops of secondary antibody solution was added onto the tissue section and allowed to stand for 30 minutes. After washing twice with 1 × PBS, a DAB solution (DAB substrate kit by Nichirei Biosciences Co., Ltd., with addition and mixing of reagents A and B one drop at a time to 1 mL of Elix water, followed by addition and mixing of reagent C one drop at a time) (for DAB staining) was added at 150 µL onto the tissue section, and after antigen-antibody reaction, it was observed under a microscope. For fluorescent staining, one drop of encapsulation material was added onto the tissue section, and after covering with cover glass, it was observed under a microscope.

### <Primary antibody (dilution factor)-secondary antibody combinations>

· Anti-mCherry antibody (Abcam, 1/500)-anti-rabbit IgG antibody (Nichirei Biosciences, Inc.)
· anti-Synaptophysin antibody (Abcam, 1/500)-anti-rabbit IgG antibody (Nichirei Biosciences, Inc.)
· Anti-Chromogranin A antibody (DAKO, 1/500)-anti-rabbit IgG antibody (Nichirei Biosciences, Inc.)
· anti-Ki67 antibody (Abcam, 1/200)-anti-rabbit IgG antibody (Nichirei Biosciences, Inc.)
· Anti-Insulin antibody (DAKO) - anti-guinea pig IgG antibody (Biotium Co.)
· Anti-Somatostatin antibody (Santa Cruz Co., 1/300)-anti-mouse IgG antibody (Biotium Co.)
· anti-Glucagon antibody (Santa Cruz Co., 1/300)-anti-mouse IgG antibody (Biotium Co.)

### (viii) RNA recovery, RNA extraction and cDNA synthesis

For RNA recovery, the cultured cells were washed with PBS (-) (Nakalai Tesque) and the cells were lysed with 350 µL of LBP buffer. NucleoSpin^{R} RNA Plus (Takara) was used for RNA extraction, according to the recommended protocol. For cDNA synthesis, a Primescript-main-strand cDNA synthesis kit (Takara) was used according to the recommended protocol.

### (ix) qRT-PCR

GoTaq qPCR master mix (Promega) was used according to the recommended protocol. Analysis was performed using a StepOne Plus System (Life Technologies). The primers and PCR reaction conditions used were the following.

### [Table 1]

**Table 1.**

| (a) Primers | | | |
|---|---|---|---|
| Target gene | Fw/Fv | Length (mer) | Sequence (5' → 3') |
| β-actin | Fw | 20 | GCCAACCGTGAAAAGATGAC (SEQ ID NO: 5) |
| | Rv | 19 | TCCGGAGTCCATCACAATG (SEQ ID NO: 6) |
| Mycl | Fw | 20 | ACGGCACTCCTAGTCTGGAA (SEQ ID NO: 7) |
| | Rv | 21 | CCACGTCAATCTCTTCACCTT (SEQ ID NO: 8) |
| Fev | Fw | 19 | CCGTCGGAGATGGTCTTTT (SEQ ID NO: 9) |
| | Rv | 20 | CCAGGAGAAACTGCCACAAC (SEQ ID NO: 10) |
| Cck | Fw | 20 | ACTGCTAGCGCGATACATCC (SEQ ID NO: 11) |
| | Rv | 20 | TATTCTATGGCTGGGGTCCA (SEQ ID NO: 12) |
| Pax4 | Fw | 20 | GTACTTCGGGCACTTCAGGA (SEQ ID NO: 13) |
| | Rv | 20 | TTACTGTGGGGACTGGGAAG (SEQ ID NO: 14) |

### (b) PCR reaction conditions

· 95°C, 2 minutes
· 95°C (15 seconds), 60°C (1 min) [40 cycles]
· 95°C (15 seconds), 60°C (1 min), 95°C (15 seconds)

### (x) Isolation of pancreatic islets

Somnopentyl (Kyoritsu Seiyaku Corp.) was intraperitoneally injected into 8-week-old KH2-Mycl mice for anesthesia. After laparotomy, the orifice of the common bile duct of the duodenum was identified and the upper part of the common bile duct and the intestinal tract were occluded with a bulldog clamp. A cut line was formed in the common bile duct at the border with the duodenum, and 2 mL of M199 medium (Gibco) containing Colalgenase P (Roche) (2 mg/mL) was injected. The pancreas was the extracted and transferred to a 50 mL tube, and digestion treatment was carried out for 14 minutes and 30 seconds in a hot water bath at 37°C. This was suspended in 25 mL of 10% FBS-containing cold M199 medium and centrifuged twice (1000 rpm, 4°C, 2 minutes). The supernatant was discarded and the precipitate was suspended in 10 mL of Histopaque (Sigma), after which 10 mL of cold M199 medium containing 10% FBS was poured in and the mixture was centrifuged (1000 rpm, 4°C, 30 minutes). The supernatant was transferred to a separate 50 mL tube, 25 mL of 10% FBS-containing M199 medium was further added, and then the pancreatic islets were separated by density gradient centrifugal separation using Lymphoprep (1000 rpm, 4°C, 2 minutes).

### (xi) Dispersion of pancreatic islets

The isolated pancreatic islets were sampled in a 1.5 mL silicon tube, and 100 µL of dispersion buffer (see Table 2) was added. The mixture was allowed to stand at 37°C for 15 minutes and then dispersed by pipetting.

**[Table 2]**

| Table 2. Dispersion buffer | |
|---|---|
| Solution 1 | 10 mL |
| Solution 2 | 10 mL |
| 0.1M EGTA | 4 mL |
| Hepes | 0.1909 g |
| BSA | 0.04 g |
| H₂O | 20 mL |

(adjusted to pH 7.4)

### [Table 3]

**Table 3.**

| | |
|---|---|
| Solution 1 | 0.64M NaCl |
| Solution 2 | 20 mM KCl |
| | 40 mM NaHCO3 |
| | 10 mM MGgCl₂-6H₂O |

### (x) Culturing of mouse primary islet cells

The isolated pancreatic islets were transferred to a 1.5 mL tube and the pancreatic islet cells were separated with TrypLE. After washing with RPMI medium, the cell count was measured. After seeding pancreatic islet cells in a 96-well plate, 30 µL of Matrigel was added onto them. The cells and Matrigel were mixed before a Pipetman, and the 96-well plate was allowed to stand in a CO₂ incubator at 37°C for 30 minutes for crosslinking of the gel. Next, 120 µL of medium was added and culturing was carried out at 37°C, 5% CO₂. Observation and imaging of pancreatic islet cells was carried out using a KEYENCE BZ-710.

### (xi) Subculturing of pancreatic islet cells

A 120 µL portion of TrypLE was added to the Matrigel in which the pancreatic islet cells were being cultured, and the mixture was pipetted to disrupt the gel. The pancreatic islet cells were transferred to a 1.5 mL tube, and then 300 µL of TrypLE was further added and the mixture was allowed to stand in a CO₂ incubator for 10 minutes at 37°C. This was followed by centrifugal separation at 200 g for 2 minutes and washing with RPMI medium, after which the cell count was measured. Pancreatic islet cells were seeded in a 96-well plate and subsequently subjected to three-dimensional culturing with Matrigel. Subculturing of the pancreatic islet cells was carried out once per week, using as the culture solution RPMI 1640 (Nacalai Tesque; 09892-15) with addition of a 45 w/v% D(+)-glucose solution (Wako; 079-05511) to an appropriate final glucose concentration (such as 4.5 g/L, 4.5 g/L).

### (xii) Cell fate tracking experiment

A 0.2 mL portion of tamoxifen (20 mg/mL) was intraperitoneally administered 5 times per week to 8- to 12-week-old mice with a trackable cell lineage (KH2-Mycl; Ins1/Gcg/Sst-CreERT2; R26-mTmG). One week later, pancreatic islets were isolated from the mice. After separating the pancreatic islet cells with TrypLE, they were seeded in AggreWell at 1.0 × 10⁵ cells/well. After adding 4-OHT (100 nmol/mL) to the culture solution, culturing was continued for 2 days. The pancreatic islet cells were collected and three-dimensional culturing was carried out with Matrigel. Another week later, the proportion of GFP-positive cells was evaluated by imaging using a KEYENCE BZ-710, and FACS.

### (xiii) Pancreatic islet cell grafting experiment

Immunodeficient mice (NOD/ShiJic-scid Jcl) were intraperitoneally administered streptozotocin (100 mg/kg) 3 times per week to induce diabetes. Pancreatic islet cells (5 × 10⁵ cells) that had been subcultured 18 times beneath the renal capsule of the mice were grafted using a Hamilton syringe. The blood glucose levels were measured each week, measuring non-fasting blood sugar.

### (ix) Intraperitoneal glucose tolerance test (IPGTT)

The Mycl-expressing group and the control group were starved for 12 to 16 hours. The body weight of each mouse was then measured, and a D-glucose solution was intraperitoneally injected into the mice to a D-glucose concentration of 2 g/kg (per mouse). After 15, 30, 60 and 120 minutes, blood was collected from each mouse through the tail and the blood glucose level was measured using an Antsense meter (Horiba).

### Example 1: Activation of Mycl-induced pancreatic islet cell proliferation during testing with glucose

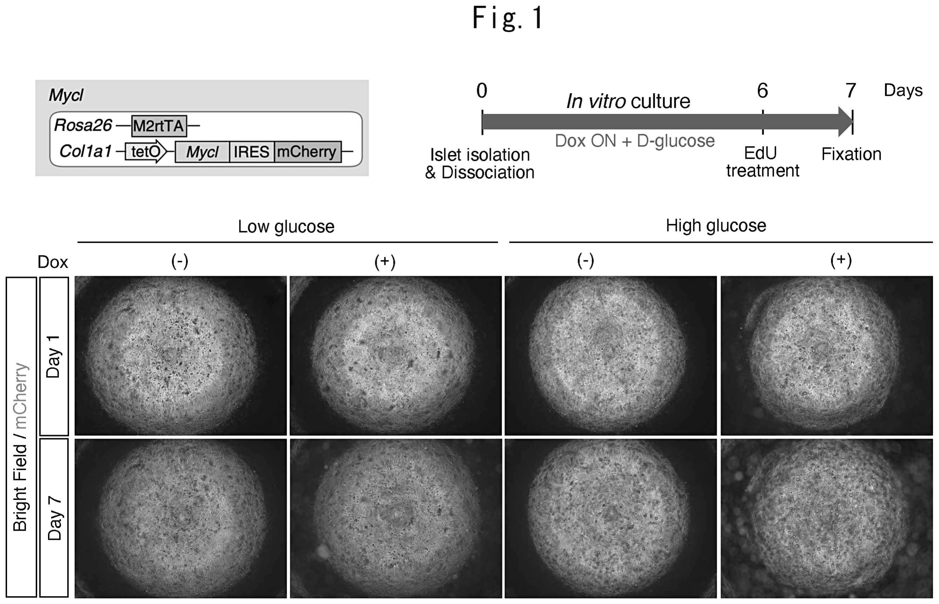

Expression of the Mycl gene can be confirmed by expression of the mCherry gene incorporated downstream from the gene. The mCherry gene is a gene coding for red fluorescent protein, and expression of the gene causes cells to exhibit a red color. The glucose concentration in the medium was varied, and the difference in Mycl-induced pancreatic islet cell proliferation activity was examined. it was found that culturing conditions with high-glucose medium (D-glucose; 4.5 g/L) increases efficiency of Mycl-expressing pancreatic islet cell proliferation, compared to culturing conditions with low-glucose medium (D-glucose; 1.0 g/L) (see Fig. 1).

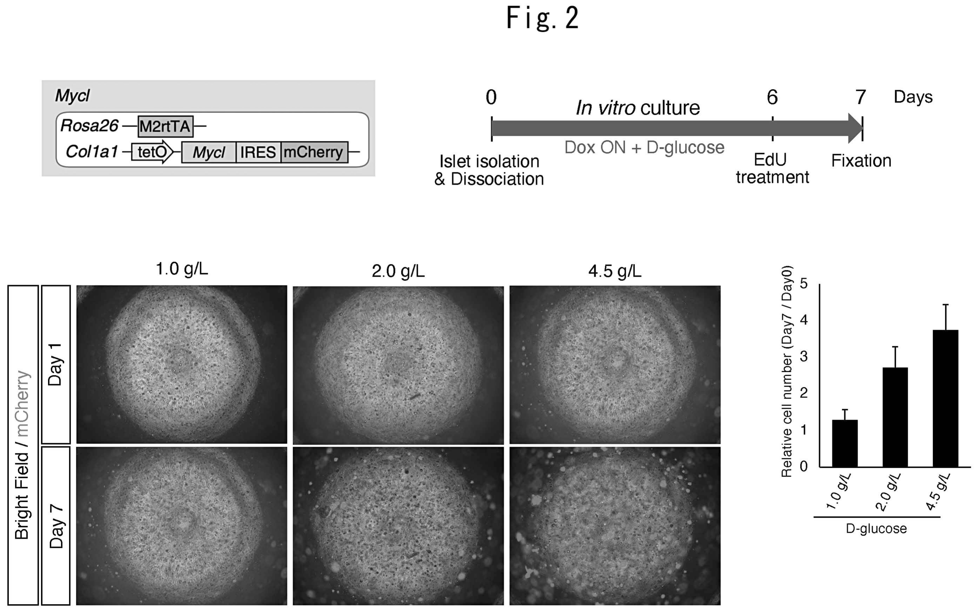

It was examined whether or not Mycl-induced pancreatic islet cell proliferation is dependent on glucose concentration. Mycl-induced pancreatic islet cells were cultured in the same manner as described above with medium glucose concentrations of 1.0, 2.0 and 4.5 g/L, and the culturing efficiency was evaluated. Examination of mCherry expression and comparative cell counts showed that efficiency of Mycl-expressing pancreatic islet cell proliferation increases in a glucose concentration-dependent manner (see Fig. 2).

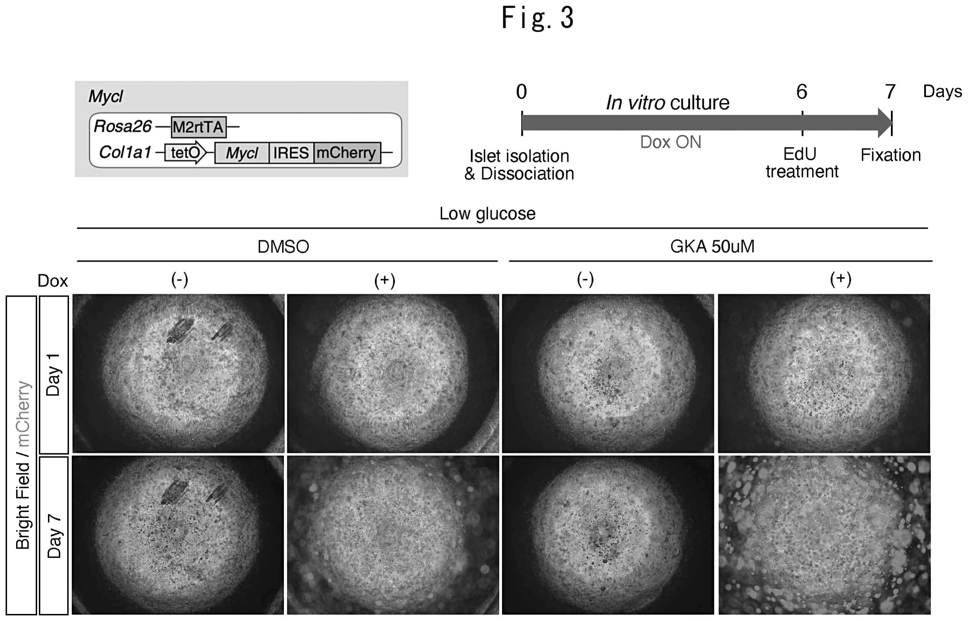

Chemical agent-induced change in efficiency of Mycl-expressing pancreatic islet cell proliferation was examined using an agent that activates glucokinase, an enzyme associated with glucose metabolism (glucokinase activator; GKA). As shown in Fig. 3, addition of GKA (50 µM) increased efficiency of Mycl-expressing pancreatic islet cell proliferation compared to no addition (DMSO addition).

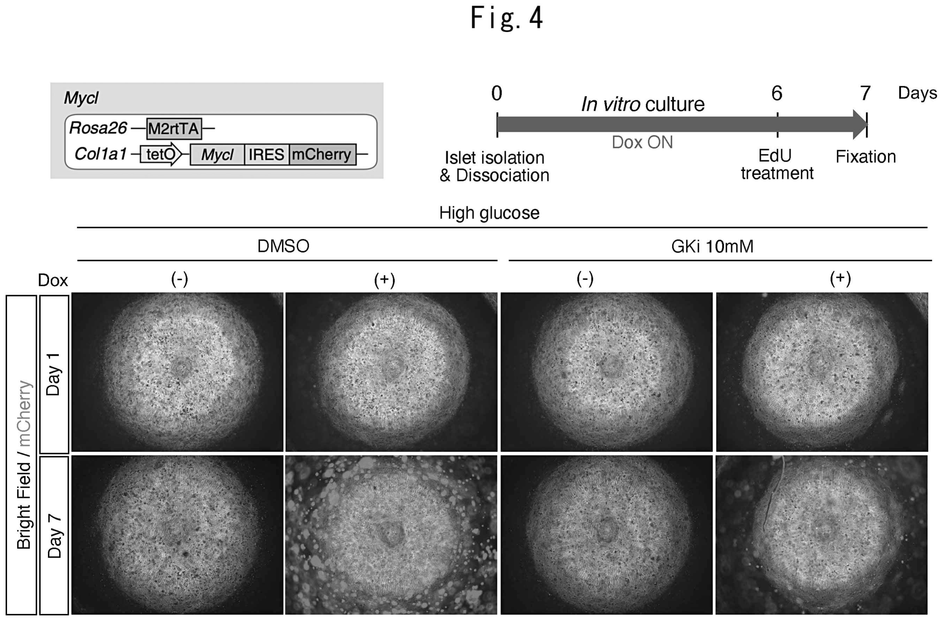

Chemical agent-induced change in efficiency of Mycl-expressing pancreatic islet cell proliferation was next examined using an agent that inhibits activation of glucokinase, an enzyme associated with glucose metabolism (glucokinase inhibitor; GKI). As shown in Fig. 4, addition of GKI (10 mM) decreased efficiency of Mycl-expressing pancreatic islet cell proliferation compared to no addition (DMSO addition).

### Example 2: Glucose-induced activation of Mycl-induced pancreatic islet cell proliferation in vivo

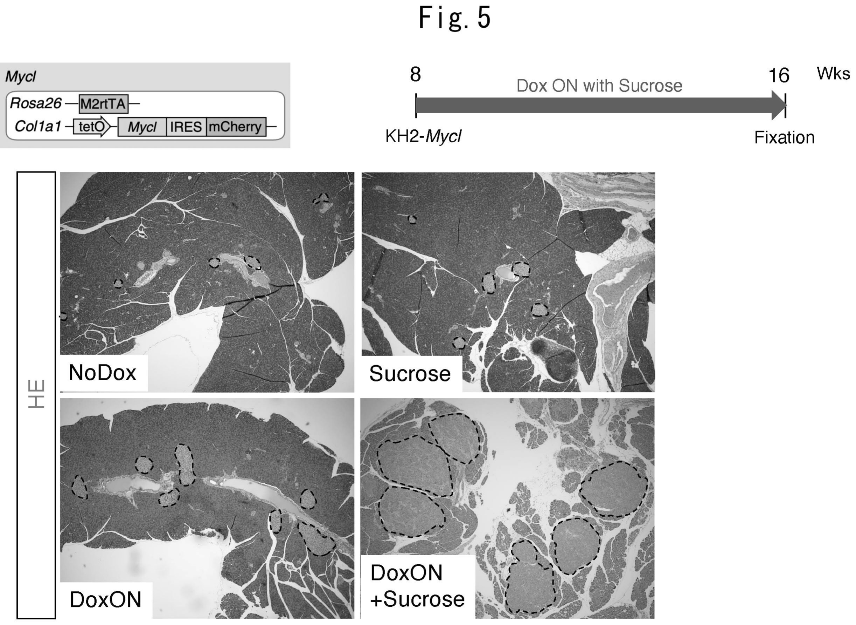

Mice with trackable pancreatic islet cells (Ins1-ires-CreERT2; Gcg-CreERT2, Sst-ires-CreERT2) were used to examine glucose-induced activation of Mycl-induced pancreatic islet cell proliferation *in vivo.* In Example 1 (*ex vivo*)*,* glucose was added to the medium, but for this Example, sucrose (glucose + fructose) was used instead of glucose. Pancreatic islet cell proliferation activity was evaluated by immunostaining. The results in Fig. 5 show that in the Dox induction + sucrose administration system, notable proliferation of pancreatic islet cells was observed compared to the other experiment systems. This demonstrated that pancreatic islet proliferation mediated by the Mycl gene is promoted by administration of sucrose (glucose + fructose) even in the body.

### Example 3: Effect on Mycl-induced pancreatic islet cell proliferation activity by continuous culturing in the presence of glucose

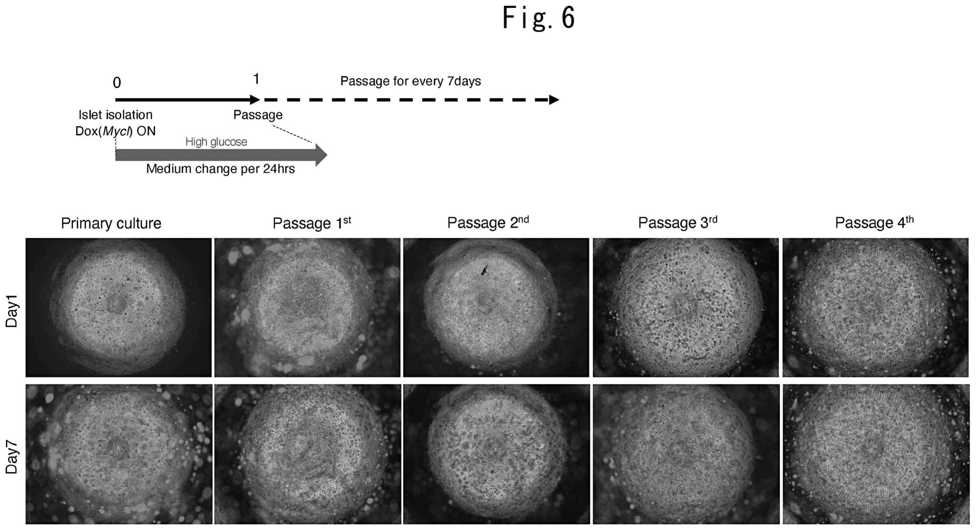

The effect of continuously present glucose in the medium on Mycl-induced pancreatic islet cell proliferation activity was examined. A high glucose concentration was used for the culturing. The cells were subcultured for a total of 4 cycles, with 7 days of culturing as one cycle. Activation of cell proliferation due to Mycl induction was found to be reduced by repeated subculturing (see Fig. 6). This indicated that continuous culturing impairs the ability of Mycl-expressing pancreatic islet cells to proliferate even under culturing conditions with high-glucose concentration, suggesting that "glucotoxicity" is also involved.

### Example 4: Continuous proliferation of Mycl-induced pancreatic islet cells by adjustment of glucose concentration

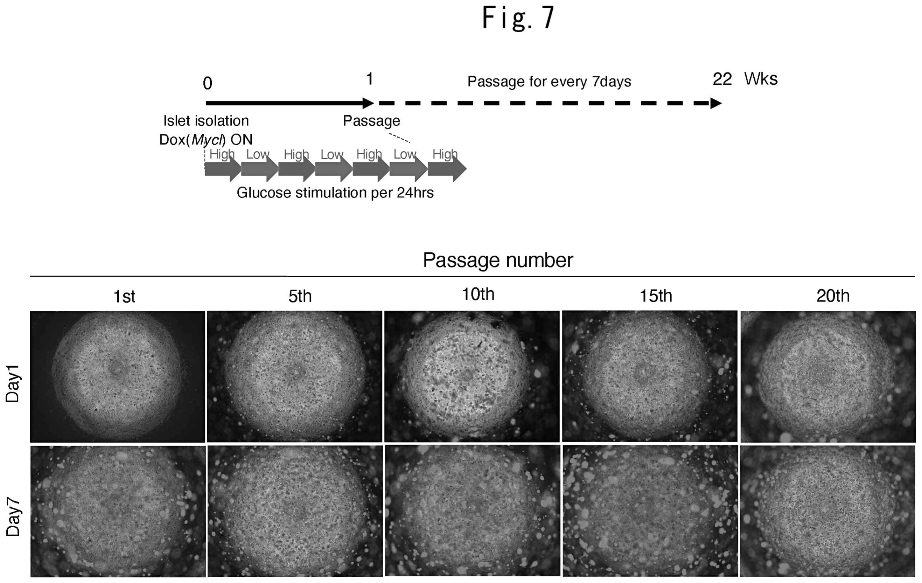

Since continuous culturing with a high-glucose concentration medium suggested that "glucotoxicity" is involved, as demonstrated in Example 3, it was examined whether or not prolonged and continuous culturing of cells is possible by adjusting the glucose concentration. High glucose concentration and low glucose concentration conditions were alternated every 24 hours and activation of cell proliferation was examined. As shown in Fig. 7, Mycl-induced pancreatic islet cell proliferation activity was sustained for at least 20 subcultures (approximately 22 weeks) under such culturing conditions. It was thus demonstrated that repeatedly alternating high-glucose/low-glucose (hereinafter referred to as "High-Low glucose") can maintain high proliferation potency for Mycl-induced pancreatic islet cells, and allows sustained induction of proliferation.

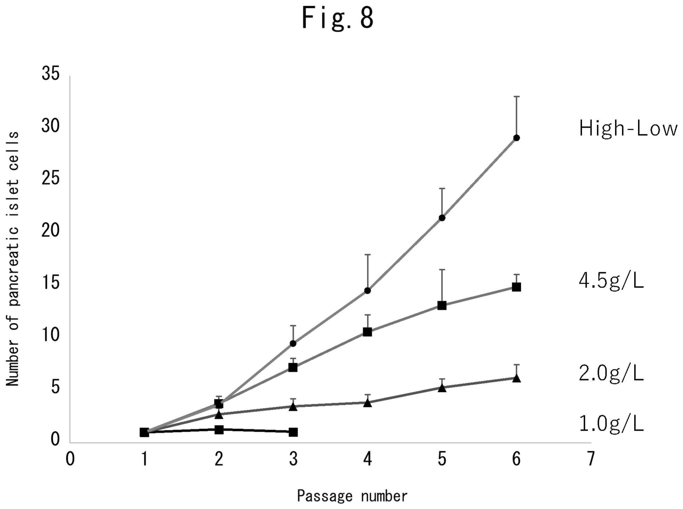

The number of Mycl-induced pancreatic islet cells in the High-Low glucose culturing system was counted to determine the time-dependent change in cell proliferation. As shown in Fig. 8, repeating high-glucose/low-glucose conditions maintains high proliferation potency for Mycl-induced pancreatic islet cells and allows sustained induction of proliferation.

### Example 5: Pancreatic islet cell tracking test

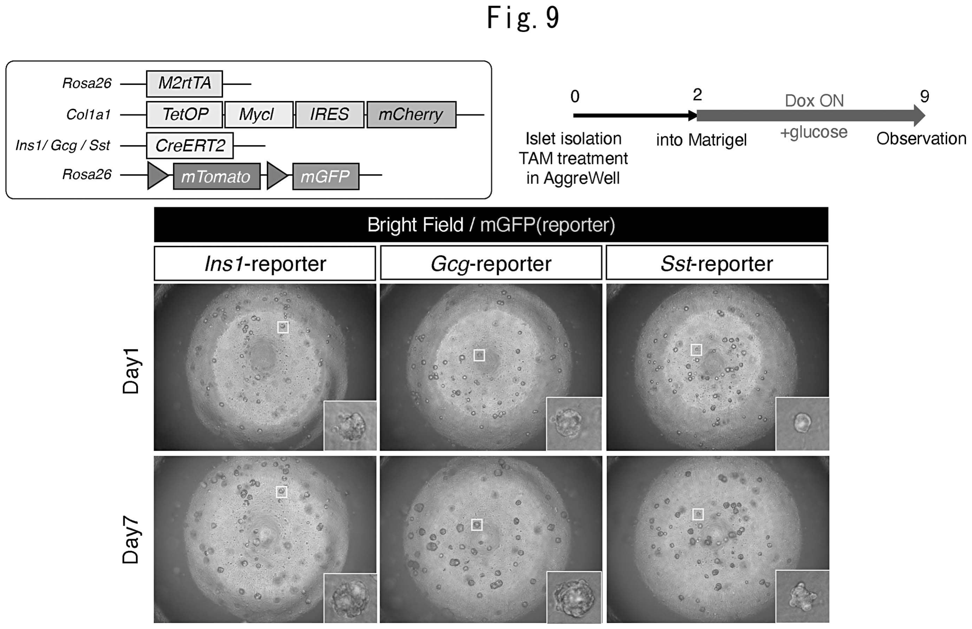

A test was conducted to track expression of the Ins1 gene which is specifically expressed in pancreatic β cells, the gene Gcg gene which is specifically expressed in α cells and the Sst gene which is specifically expressed in δ cells. Ins1-positive cells (β cells), Gcg-positive cells (α cells) and Sst-positive cells (δ cells) were labeled by a common method, and it was examined whether or not each of the cell types proliferated. More specifically, the expression level of each gene was compared based on expression of the reporter gene mGFP (see Fig. 9). The data in Fig. 9 show that the Mycl gene promotes proliferation of mature hormone-producing cells, namely β cells, α cells and δ cells.

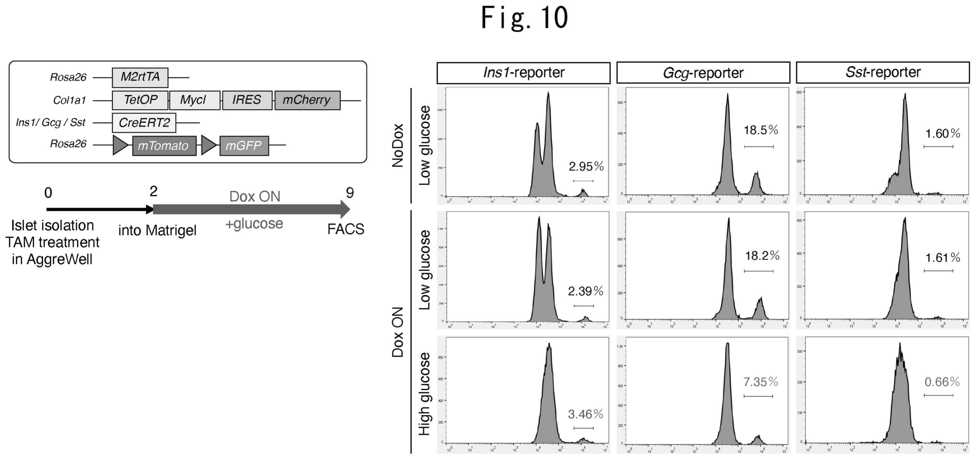

Upon further examining the proliferation efficiency of mature hormone-producing cells by FACS analysis, it was found that culturing with the Mycl gene and with high glucose concentration resulted in more efficient proliferation of Ins1-positive cells (β cells) (Fig. 10).

### Example 6: Recovery of diabetic mice

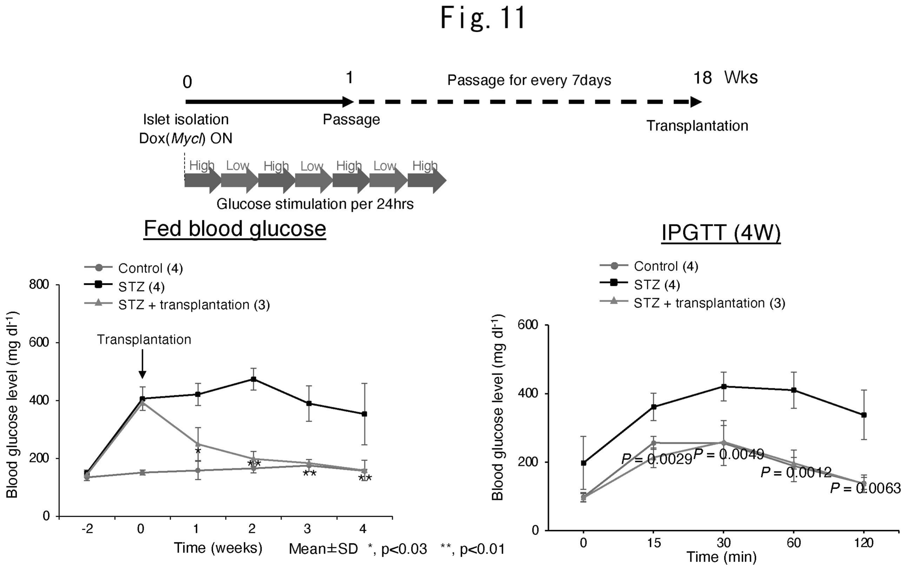

Change in blood glucose levels of diabetic mice by prolonged growth of Mycl-induced pancreatic islet cells was examined under High-Low glucose conditions (alternatingly repeated high-glucose/low-glucose conditions). As shown in Fig. 11, transplanting Mycl-induced pancreatic islet cells into diabetic mice resulted in successful normalization of blood glucose levels. It was thus confirmed in intraperitoneal glucose tolerance testing (IPGTT) that glucose tolerance was improved and that pancreatic islet cells grown for prolonged periods exhibited high function.

### <Summary>

Based on the results of the Examples, first, it was found that the proliferative activity of mature pancreatic islet cells by induction of the Mycl gene is dependent on glucose concentration (see Fig. 1 and Fig. 2). In fact, proliferation of pancreatic islet cells was accelerated by glucokinase activator (GKA) that activates glucokinase, a molecule in the intracellular metabolic pathway for glucose, while proliferation of pancreatic islet cells was inhibited by an inhibitor, namely glucokinase inhibitor (GKI) (see Fig. 3 and Fig. 4). It was shown that, even *in vivo,* expression of the Mycl gene in combination with addition of glucose strongly promotes proliferation of pancreatic islet cells (see Fig. 5). It was also found, on the other hand, that continuous culturing under high-glucose culturing conditions lowers cell proliferative activity due to glucotoxicity (see Fig. 6). Repeated high-glucose conditions and low-glucose conditions, in order to avoid glucotoxicity, was able to successfully maintain high proliferative activity, and the induced growth was sustainable for at least six months (see Fig. 7 and Fig. 8). Proliferation of mature β cells was promoted by high-glucose conditions (see Fig. 9 and Fig. 10). The continuously amplified pancreatic islet cells produced insulin in response to glucose in the body, lowering blood glucose in the animal diabetes model. A high level of such functionality was demonstrated (see Fig. 11).

### INDUSTRIAL APPLICABILITY

According to the invention it is possible to induce proliferation of pancreatic islet cells in a continuous manner, and therefore the invention is expected to aid in curing diabetes by pancreatic islet cell grafting. In addition, activating the Mycl gene together with glucose *in vivo* allows efficient amplification of pancreatic islet cells in the body, and this is expected to aid in curing of diabetes by gene therapy.

All of the publications and patent literature cited herein are incorporated into the present specification in their entirety as reference. The specific embodiments of the invention were explained in the present specification for the purpose of example, and it will be readily appreciated by a person skilled in the art that various modifications may be employed such as are not outside of the spirit and scope of the invention.

[Sequence Listing]

## Claims

1. A method of promoting and/or sustaining proliferation activity for pancreatic islet cells into which the Mycl gene or its gene product has been transferred or where its expression has been induced, using an activator of the glucose-stimulated activation pathway.

2. The method according to claim 1, wherein the activator of the glucose-stimulated activation pathway is at least one selected from the group consisting of glucose, sucrose and glucokinase activators.

3. The method according to claim 1 or 2, wherein the Mycl gene comprises:
(1) a nucleic acid comprising the nucleotide sequence represented by SEQ ID NO: 1 or 3; or
(2) a nucleic acid encoding a polypeptide which hybridizes with a nucleic acid comprising the nucleotide sequence represented by SEQ ID NO: 1 or 3 under stringent conditions, and which has a function of sustaining and/or promoting the proliferative activity of pancreatic islet-like cells when expression of the Mycl gene has been induced.

4. The method according to claim 1 or 2, wherein the Mycl gene product comprises:
(1) a polypeptide comprising the amino acid sequence represented by SEQ ID NO: 2 or 4; or
(2) a polypeptide having at least 80%, 85%, 90%, 95%, 97%, 98% or 99% sequence identity with the amino acid sequence represented by SEQ ID NO: 2 or 4, and having the function of sustaining and/or promoting the proliferative activity of pancreatic islet-like cells

5. The method according to any one of claim 1 to 4, wherein the Mycl gene is transiently expressed.

6. The method according to any one of claim 1 to 5, wherein high-concentration glucose and low-concentration glucose are alternately applied.

7. The method according to any one of claim 1 to 6, wherein the pancreatic islet cells are derived from primary islet cells isolated from pancreas, cultured islet cells or stem cells.

8. The method according to claim 7, wherein the stem cells are selected from the group consisting of iPS cells, ES cells and somatic stem cells.
